# EUROPÄISCHE PATENTSCHRIFT

(11) **EP 2 926 734 B1**
(45) Veröffentlichungstag und Bekanntmachung des Hinweises auf die Patenterteilung: **26.06.2019**
(21) Anmeldenummer: 14001228.7
(22) Anmeldetag: 02.04.2014
(51) Int. Cl.: A61B 6/04, A61B 6/00

(54) **Verfahren zur Einrichtung einer Patientenbestrahlungseinrichtung**
Method for setting up a patient irradiation device
Procédé de montage d'un dispositif d'irradiation de patient

(43) Veröffentlichungstag der Anmeldung: 07.10.2015
(73) Patentinhaber: BEC GmbH, 72793 Pfullingen (DE)
(72) Erfinder: Buck, Matthias, 72764 Reutlingen (DE); Marianowski, Karin, 72070 Tübingen (DE); Koch, Dimitri, 72768 Reutlingen (DE)
(74) Vertreter: Reinhardt, Annette

(56) Entgegenhaltungen:
- US-A- 5 820 553
- US-A1- 2002 085 668
- US-A1- 2002 122 530
- US-A1- 2005 281 374
- US-A1- 2008 031 414
- US-A1- 2008 289 106
- US-A1- 2009 144 902
- US-A1- 2009 238 338
- US-A1- 2013 053 702
- ZULLO ET AL: "Implication of CT Table Sag on Geometrical Accuracy During Virtual Simulation", MEDICAL DOSIMETRY, ELSEVIER, US, Bd. 32, Nr. 4, 1. November 2007 (2007-11-01), Seiten 233-236, XP022323085, ISSN: 0958-3947, DOI: 10.1016/J.MEDDOS.2007.07.001

## Beschreibung

Die Erfindung betrifft ein Verfahren zur Einrichtung einer Patientenbestrahlungseinrichtung der im Oberbegriff des Anspruchs 1 angegebenen Gattung.

Aus der EP 1 985 237 A1 ist eine Patientenbestrahlungseinrichtung bekannt, die eine Patientenpositioniereinrichtung, eine Strahlenquelle und ein Bildgebungssystem umfasst. Zur Erfassung mindestens einer Information über die Position der Patientenaufnahme im Raum ist ein Positionserfassungssystem vorgesehen, das die Position der Patientenaufnahme beispielsweise optisch oder über Funk erfasst. Zusätzlich kann an der Patientenaufnahme eine Röntgenmarke vorgesehen sein, die auf einer Röntgenaufnahme erkennbar ist.

Aus der US 7,695,192 B2 ist ein Bildgebungssystem bekannt, das über einen Roboterarm im Raum beweglich gehalten und im Raum positionierbar ist.

Aus der US 2002/0085668 A1 geht ein Verfahren zur Positionierung eines Patienten an einer Patientenbestrahlungseinrichtung hervor, bei dem zur Einrichtung der Bestrahlungseinrichtung ein Kalibrierphantom an einem Röntgendetektor angeordnet wird. Das Kalibrierphantom besitzt Trackingmarker, über die die Position des Phantoms durch ein Trackingsystem ermittelt wird. Anschließend wird das Kalibrierphantom über ein Bildgebungssystem abgebildet. Nach der Zuordnung der Position des Phantoms im Raum zur erzeugten Abbildung erfolgt die Positionierung des Patienten über eine bewegliche Patientenliege. Dabei wird die Patientenliege mit Hilfe des Trackingsystems sowie Markern am Patienten oder der Patientenliege bewegt.

Aus der US 2008/0031414 A1 ist ein System zur Ermittlung der Position einer Patientenliege an einem CT oder einer Bestrahlungseinrichtung bekannt, bei dem die Patientenliege im Raum beweglich ist. Die Bestrahlungseinrichtung ist fest im Raum montiert. Ein im Raum bewegbares Bildgebungssystem ist nicht vorgesehen.

Bei der US 2002/0122530 A1 ist ein Verfahren zur Kalibrierung eines Computertomographen mit einem Kalibrierphantom bekannt. Die Position des Kalibrierphantoms wird über Infrarotkameras ermittelt. Außerdem wird das Phantom vom CT abgebildet. Auch die Position des CTs selbst im Raum kann ermittelt werden. Eine Ermittlung der Position einer Patientenaufnahme ist jedoch nicht vorgesehen.

Der Erfindung liegt die Aufgabe zugrunde, ein Verfahren zur Einrichtung einer Patientenbestrahlungseinrichtung zu schaffen, mit dem die Bestrahlung eines Zielvolumens sehr genau erfolgen kann.

Bezüglich des Verfahrens zur Einrichtung einer Patientenbestrahlungseinrichtung wird diese Aufgabe durch ein Verfahren mit den Merkmalen des Anspruchs 1 gelöst. Es ist bekannt, bei beweglichen Bildgebungssystemen und bei beweglichen Patientenaufnahmen die Position des Bildgebungssystems bzw. der Patientenaufnahme über die Ansteuerung der Antriebe für das Bildgebungssystem bzw. die Patientenpositioniereinrichtung zu erfassen. Es hat sich allerdings gezeigt, dass die hierdurch erreichbare Genauigkeit unzureichend sein kann, da zwar die Position des Bildgebungssystems über die Mittel zur Erfassung mindestens einer Information über die Position des Bildgebungssystems, beispielsweise über die Antriebe des Bildgebungssystems, näherungsweise bekannt ist, die tatsächliche Lage des von dem Bildgebungssystem abgebildeten Objekts jedoch nicht ausreichend genau bekannt ist, da beispielsweise die Lage der bildgebenden Einrichtungen des Bildgebungssystems in einem Gehäuse des Bildgebungssystems Toleranzen unterliegt.

Um auf einfache Weise sowohl die Lage der Patientenaufnahme als auch die Lage einer von dem Bildgebungssystem aufgenommenen Abbildung zu ermitteln, ist vorgesehen, dass zur Einrichtung der Patientenbestrahlungseinrichtung in mindestens einer Position von Patientenaufnahme und Bildgebungssystem die Position des mindestens einen ersten Positionsreferenzelements von dem Positionserfassungssystem ermittelt wird, das Bildgebungsreferenzelement mit dem Bildgebungssystem abgebildet wird, die Position des Bildgebungsreferenzelements ermittelt und dieser Position des Bildgebungssystems, des Bildgebungsreferenzelements und des ersten Positionsreferenzelements mindestens eine Information zur Position der mit dem Bildgebungssystem von dem Bildgebungsreferenzelement erzeugten Abbildung zugeordnet wird. Die Information über die Position der mit dem Bildgebungssystem erzeugten Abbildung kann beispielsweise die Koordinaten des Bildgebungsreferenzelements in einem Koordinatensystem des Bildgebungssystems umfassen. Die Information über das erste Positionsreferenzelement und das Bildgebungsreferenzelement kann beispielsweise Koordinaten in einem Raumkoordinatensystem oder in einem bewegten Koordinatensystem eines der bewegten Elemente sein. Die Information zur Position kann auch eine Information sein, die indirekt einen Rückschluss auf die Position eines Elements oder einer Abbildung erlaubt.

Dadurch, dass die Patientenbestrahlungseinrichtung für mindestens eine Position von Patientenaufnahme und Bildgebungssystem Informationen über die Position des ersten Positionsreferenzelements, über die Position des Bildgebungsreferenzelements und über die Position der von dem Bildgebungsreferenzelement erzeugten Abbildung besitzt, kann ausgehend von dieser mindestens einen Position anhand einer aktuellen Position des ersten Positionsreferenzelements und anhand der Bewegung des Bildgebungssystems ausgehend von dieser mindestens einen Position die Lage einer von dem Bildgebungssystem erzeugten Abbildung mit hoher Genauigkeit ermittelt werden. Es kann auch vorgesehen sein, die Position des Bildgebungssystems jeweils zu bestimmen, beispielsweise mittels mindestens eines an dem Bildgebungssystem angeordneten dritten Positionsreferenzelements oder dgl..

Die Position des mindestens einen Bildgebungsreferenzelements wird vorteilhaft anhand der relativen Lage des mindestens einen Bildgebungsreferenzelements zu dem mindestens einen ersten Positionsreferenzelement ermittelt. Die relative Lage des Bildgebungsreferenzelements zu dem ersten Positionsreferenzelement kann beispielsweise mit einem mechanischen Messsystem wie beispielsweise einer Koordinatenmessmaschine wie einem Messarm oder mit einem optischen Messsystem ausgemessen werden. Es kann auch vorgesehen sein, dass das Bildgebungsreferenzelement in einer Aufnahme in bekanntem Abstand zu dem ersten Positionsreferenzelement angeordnet wird oder das Bildgebungsreferenzelement in der Patientenaufnahme in festem, bekanntem Abstand zu dem ersten Positionsreferenzelement angeordnet ist.

Alternativ kann mindestens ein zweites Positionsreferenzelement in bekannter relativer Lage zu dem mindestens einen Bildgebungsreferenzelement vorgesehen sein, und die Position des mindestens einen Bildgebungsreferenzelements kann über eine von dem Positionserfassungssystem ermittelte Position des mindestens einen zweiten Positionsreferenzelements ermittelt werden. Hierzu kann beispielsweise bei der Einrichtung der Patientenbestrahlungseinrichtung ein Kalibrierelement an der Patientenaufnahme angeordnet werden, das sowohl das Bildgebungsreferenzelement als auch das zweite Positionsreferenzelement aufweist. Dadurch, dass die Lage des Kalibrierelements über das mindestens eine zweite Positionsreferenzelement ermittelt wird, sind an die Positionierung des Kalibrierelements an der Patientenaufnahme keine hohen Anforderungen zu stellen. Vorzugsweise kann das Kalibrierelement beliebig auf der Patientenaufnahme angeordnet werden.

Erste Positionsreferenzelemente sind dabei an der Patientenaufnahme angeordnete Positionsreferenzelemente und zweite Positionsreferenzelemente sind Positionsreferenzelemente, die an einem Kalibrierelement angeordnet sind. Dritte Positionsreferenzelemente sind Positionsreferenzelemente, die an dem Bildgebungssystem angeordnet sind.

Ein besonders einfacher Aufbau ergibt sich, wenn das mindestens eine erste Positionsreferenzelement und das mindestens eine Bildgebungsreferenzelement als mindestens ein von dem Bildgebungssystem abbildbares und von dem Positionserfassungssystem erfassbares, kombiniertes Referenzelement ausgebildet sind und die Position des mindestens einen kombinierten Referenzelements über das Positionserfassungssystem ermittelt wird. Das kombinierte Referenzelement kann an der Patientenaufnahme in dem vom Bildgebungssystem abbildbaren und vom Positionserfassungssystem erfassbaren Bereich beliebig angeordnet werden, da die Ermittlung der Position über das Positionserfassungssystem erfolgt.

Vorteilhaft ermittelt das Positionserfassungssystem mindestens eine Information über die Orientierung des mindestens einen Positionsreferenzelements im Raum. Das Positionsreferenzelement ist insbesondere so ausgebildet, dass das Positionserfassungssystem die absolute Position, also die Koordinaten des Positionsreferenzelements im Raum, und die absolute Orientierung, also die Drehlage des Positionsreferenzelements im Raum, ermitteln kann. Vorteilhaft ermittelt das Bildgebungssystem mindestens eine Information über die Orientierung des mindestens einen Bildgebungsreferenzelements relativ zu dem Bildgebungssystem. Vorzugsweise ermittelt das Bildgebungssystem Informationen über die Position des Bildgebungsreferenzelements im Raum und über die Drehlage des Bild-gebungsreferenzelements um alle drei Raumachsen. Über das Positionsreferenzelement und das Bildgebungsreferenzelement lässt sich dadurch nicht nur deren Position, sondern auch deren Orientierung, also deren Drehlage um alle Raumachsen, ermitteln.

Ein einfacher Aufbau ergibt sich, wenn das Positionserfassungssystem mindestens eine Kamera umfasst und die Positionsreferenzelemente fotogrammetrische Marken sind. Es hat sich gezeigt, dass die Erfassung der fotogrammetrischen Marken mit einer Kamera mit sehr hoher Genauigkeit erfolgen kann. Die Kalibrierung des Positionserfassungssystems erfolgt vorteilhaft mittels eines Lasertrackingsystems. Vorteilhaft ist vorgesehen, dass die Patientenaufnahme mindestens eine Aufnahme für einen Reflektor des Lasertrackingsystems besitzt. Zur Ermittlung der Position der mindestens einen Kamera wird vorteilhaft ein Reflektor in der Aufnahme angeordnet, und in mindestens einer Position der Patientenaufnahme im Raum wird sowohl die Position des Reflektors mittels eines Lasertrackers ermittelt als auch die Anordnung des mindestens einen ersten Positionsreferenzelements mit der mindestens einen Kamera aufgenommen. Die Position des Reflektors kann dabei unmittelbar im Raumkoordinatensystem gemessen werden. Die Position des Reflektors im Raum kann jedoch auch mittelbar ermittelt werden, beispielsweise aus mit dem Lasertracker in einem anderen Koordinatensystem gemessenen Koordinaten. Die Position des ersten Positionsreferenzelements bezogen auf die Position der Aufnahme ist vorteilhaft bekannt oder wird ebenfalls mit dem Lasertrackingsystem ausgemessen. Zur Erhöhung der Genauigkeit wird vorteilhaft in mehreren Positionen der Patientenaufnahme sowohl die Position des Reflektors mittels des Lasertrackers gemessen als auch die Anordnung des mindestens einen ersten Positionsreferenzelements mit der mindestens einen Kamera aufgenommen.

Zur Einrichtung der Patientenbestrahlungseinrichtung kann auch ein Kalibrierelement an der Patientenaufnahme angeordnet werden. Das Kalibrierelement umfasst mindestens ein zweites Positionsreferenzelement, das mindestens eine von dem Bildgebungssystem erfassbare Bildgebungsreferenzelement sowie mindestens einen Reflektor des Lasertrackingsystems. Die Anordnung des zweiten Positionsreferenzelements, des Bildgebungsreferenzelements und des Reflektors an dem Kalibrierelement ist vorteilhaft bekannt. Die Anordnung kann beispielsweise durch die konstruktive Ausbildung des Kalibrierelements festgelegt sein oder genau ausgemessen werden. Zur Einrichtung der Patientenbestrahlungseinrichtung ist vorgesehen, dass die Anordnung des mindestens einen zweiten Positionsreferenzelements mit der mindestens einen Kamera aufgenommen, die Position des Bildgebungsreferenzelements mit dem Bildgebungssystem erfasst und die Position des Reflektors mittels eines Lasertrackers ermittelt wird. Die Position des Reflektors kann dabei unmittelbar im Raumkoordinatensystem gemessen werden. Die Position des Reflektors im Raum kann jedoch auch mittelbar ermittelt werden, beispielsweise aus mit dem Lasertracker in einem anderen Koordinatensystem gemessenen Koordinaten. Hieraus wird die Position des mindestens einen zweiten Positionsreferenzelements, mindestens eine Information über die Position der mit dem Bildgebungssystem von dem Bildgebungsreferenzelement erzeugten Abbildung sowie die Position der Kamera ermittelt. Die Position der Kamera kann dabei bezogen auf ein Raumkoordinatensystem oder bezogen auf ein Koordinatensystem des Lasertrackingsystems ermittelt werden. Auch ein anderes Koordinatensystem kann vorteilhaft sein. Das Kalibrierelement erlaubt auf einfache Weise, die mit dem Lasertrackingsystem, dem Positionserfassungssystem und dem Bildgebungssystem erfassten Informationen miteinander zu verknüpfen und ermöglicht dadurch eine sehr genaue Bestimmung der Position mindestens eines der Positionsreferenzelemente und der Position der mit dem Bildgebungssystem von dem Zielvolumen des Patienten erzeugten Abbildung im Betrieb. Toleranzen, die innerhalb der Einzelsysteme und zwischen den Systemen bestehen, können dadurch auf einfache Weise eliminiert werden.

Die Ermittlung der Position der Patientenaufnahme über das Positionserfassungssystem erfolgt vorteilhaft über ein Referenzpunktefeld, das aus mindestens einem von der mindestens einen Kamera aufgenommenen Bild des mindestens einen Positionsreferenzelements erzeugt wird. Das Positionsreferenzelement kann dabei ein erstes oder ein zweites Positionsreferenzelement sein. Vorteilhaft werden zur Erzeugung des Referenzpunktefelds eine Vielzahl von Bildern des mindestens einen Positionsreferenzelements in mindestens einer Position der Patientenaufnahme aufgenommen. Vorteilhaft wird in unterschiedlichen Positionen der Patientenaufnahme relativ zur Kamera eine Vielzahl von Bildern aufgenommen. Vorteilhaft sind an der Patientenaufnahme eine Vielzahl von ersten Positionsreferenzelementen vorgesehen, die vorteilhaft sowohl über die gesamte Breite als auch über die gesamte Länge der Patientenaufnahme verteilt angeordnet sind. Dadurch lässt sich ein vergleichsweise genaues Referenzpunktefeld erzeugen, so dass die Ermittlung der Position der Patientenaufnahme über die Kamera sehr genau erfolgen kann.

Die Patientenaufnahme kann beispielsweise eine Patientenliege oder dergleichen sein. Das Bildgebungssystem kann beispielsweise eine Röntgeneinrichtung oder ein MRT sein. Um im gesamten Bereich der Patientenaufnahme Aufnahmen des Patienten mit dem röntgenbasierten Bildgebungssystem machen zu können, muss die Patientenaufnahme röntgendurchlässig sein, kann also beispielsweise nicht aus Metall bestehen. Es hat sich gezeigt, dass insbesondere bei schweren Patienten eine erhebliche Durchbiegung der Patientenaufnahme erfolgen kann, die die Genauigkeit der Positionierung des Zielvolumens verschlechtert. Um den Einfluss des Patientengewichtes und damit den Einfluss der Verformung der Patientenaufnahme zu verringern, ist vorgesehen, dass zur Einrichtung der Patientenbestrahlungseinrichtung nacheinander mindestens zwei Kalibrierelemente mit unterschiedlichem Gewicht an der Patientenaufnahme angeordnet werden. Für jedes Kalibrierelement wird die relative Lage von mindestens zwei Referenzelementen zueinander ermittelt und einem dem Gewicht des Kalibrierelements entsprechenden Patientengewicht zugeordnet. Die mindestens zwei Referenzelemente sind insbesondere mindestens zwei Positionsreferenzelemente oder mindestens zwei Bildgebungsreferenzelemente. Vorteilhaft wird eine Vielzahl von Kalibrierelementen, die unterschiedliche Durchbiegungen der Patientenaufnahme bewirken, an der Patientenaufnahme angeordnet. Vorteilhaft sind die mindestens zwei Referenzelemente an dem Kalibrierelement angeordnet. Es kann jedoch auch vorgesehen sein, dass die mindestens zwei Referenzelemente an der Patientenaufnahme angeordnet sind. Vorteilhaft werden dabei Referenzelemente genutzt, die ohnehin an der Patientenaufnahme vorgesehen sind, so dass keine zusätzlichen Referenzelemente notwendig sind. Die mindestens zwei Referenzelemente besitzen dabei vorteilhaft einen möglichst großen Abstand zueinander, so dass die sich ergebende Verformung der Patientenaufnahme möglichst genau ermittelt werden kann. Die Verformung der Patientenaufnahme verändert die relative Lage der Positionsreferenzelemente zueinander und beeinflusst dadurch die vom Positionserfassungssystem ermittelte Position der Patientenaufnahme. Bei großer Durchbiegung wird diese Position verfälscht. Die Anordnung der Kalibrierelemente an der Patientenaufnahme ermöglicht die Berücksichtigung der Durchbiegung der Patientenaufnahme bei der Ermittlung der Position der Patientenaufnahme. Es kann auch vorteilhaft sein, die Verformung der Patientenaufnahme durch verschiedene Referenzpunktefelder zu berücksichtigen. Hierzu wird für jedes Kalibrierelement gemäß dem oben beschriebenen Verfahren ein Referenzpunktefeld erzeugt und einem Patientengewicht zugeordnet. Die Kalibrierelemente müssen dabei nicht zwingend Referenzelemente aufweisen. Durch Auswahl eines geeigneten Referenzpunktefelds kann dann die Durchbiegung der Patientenaufnahme bei der Positionsbestimmung berücksichtigt werden.

Um eine hohe Genauigkeit bei der Erfassung der Position der Patientenaufnahme zu erreichen, ist vorgesehen, dass das Positionserfassungssystem mindestens eine Lichtquelle umfasst, die das mindestens eine Positionsreferenzelement zumindest während einer Aufnahme anstrahlt. Die Lichtquelle kann vorteilhaft sichtbares Licht oder Infrarotlicht erzeugen. Infrarotlicht wird vom Patienten nicht wahrgenommen und kann dadurch vom Patienten nicht als störend empfunden werden.

Besonders vorteilhaft ist das Bildgebungssystem an der Patientenaufnahme beweglich gehalten. Dadurch ergibt sich ein einfacher Aufbau, und eine Kollision der Patientenaufnahme mit dem Bildgebungssystem ist vermieden. Um größere Freiheitsgrade bei der Aufnahme des Zielvolumens zu haben, kann jedoch auch vorteilhaft sein, dass das Bildgebungssystem unabhängig von der Patientenaufnahme im Raum beweglich ist. Hierzu kann beispielsweise eine separate Bewegungseinrichtung für das Bildgebungssystem vorgesehen sein.

Für ein Verfahren zur Positionierung eines Patienten an einer Patientenbestrahlungseinrichtung, wobei die Patientenbestrahlungseinrichtung eine Patientenaufnahme, eine Patientenpositioniereinrichtung, an der die Patientenaufnahme im Raum bewegbar gehalten ist, eine Strahlenquelle zur Bestrahlung eines Zielvolumens eines Patienten und ein Bildgebungssystem zur Abbildung des Zielvolumens umfasst, wobei die Patientenbestrahlungseinrichtung mindestens ein Positionserfassungssystem zur Erfassung mindestens einer Information über die Position der Patientenaufnahme im Raum umfasst, wobei das Positionserfassungssystem mindestens ein Positionsreferenzelement an der Patientenaufnahme umfasst, ist vorgesehen, dass das Bildgebungssystem in mindestens einer Dimension gegenüber der Strahlenquelle und der Patientenaufnahme im Raum bewegbar ist, wobei das Bildgebungssystem Mittel zur Erfassung mindestens einer Information über die Position des Bildgebungssystems aufweist, wobei zur Positionierung eines an der Patientenaufnahme angeordneten Zielvolumens relativ zur Strahlenquelle die Position des mindestens einen Positionsreferenzelements von dem Positionserfassungssystem ermittelt wird, das Zielvolumen mit dem Bildgebungssystem abgebildet wird und aus der Position des mindestens einen Positionsreferenzelements und mindestens einer Information zur Position der mit dem Bildgebungssystem erzeugten Abbildung die Abweichung der tatsächlichen Position des Zielvolumens zu einer Soll-Position des Zielvolumens ermittelt und die Position des Zielvolumens korrigiert wird.

Die Information über die Position der Patientenaufnahme im Raum kann dabei eine Information sein, die die Position der Patientenaufnahme im Raum direkt angibt, oder eine Information, die die Position der Patientenaufnahme indirekt, beispielsweise in einem anderen Koordinatensystem, angibt und die Rückschlüsse auf die Position im Raum zulässt, beispielsweise durch Koordinatentransformation.

Die Mittel zur Erfassung mindestens einer Information über die Position des Bildgebungssystems können beispielsweise mindestens ein drittes, an dem Bildgebungssystem angeordnetes Positionsreferenzelement umfassen. Zur Erfassung mindestens einer Information über die Position des Bildgebungssystems kann beispielsweise auch ein Antrieb des Bildgebungssystems genutzt werden.

Dadurch, dass zur Korrektur der Position des Zielvolumens sowohl die Position des mindestens einen Positionsreferenzelements als auch mindestens eine Information zur Position der mit dem Bildgebungssystem erzeugten Abbildung ermittelt wird, ist eine sehr genaue Ermittlung der Abweichung der tatsächlichen Position des Zielvolumens zu einer Soll-Position des Zielvolumens möglich.

Vorteilhaft umfasst das Positionserfassungssystem mindestens zwei Kameras, wobei die Sichtfelder der Kameras sich in einem ersten Raumbereich überlappen und die Position der Patientenaufnahme in dem ersten Raumbereich anhand der von den mindestens zwei Kameras aufgenommenen Bilder des mindestens einen Positionsreferenzelements ermittelt wird. Dadurch lässt sich die Position der Patientenaufnahme mit erhöhter Genauigkeit ermitteln.

Vorteilhaft umfasst das Positionserfassungssystem mindestens zwei Kameras, die mindestens teilweise unterschiedliche Sichtfelder aufweisen. Vorteilhaft wird die Position der Patientenaufnahme in einem nur von der ersten Kamera erfassten Raumbereich anhand mindestens eines von der ersten Kamera aufgenommenen Bildes des mindestens einen Positionsreferenzelements und in einem von der zweiten Kamera erfassten Raumbereich anhand mindestens eines von der zweiten Kamera aufgenommenen Bildes des mindestens einen Positionsreferenzelements ermittelt. Dadurch, dass die mindestens zwei Kameras mindestens teilweise unterschiedliche Sichtfelder aufweisen, lässt sich der Raumbereich, in dem die Position der Patientenaufnahme von dem Positionserfassungssystem erfassbar ist, vergrößern. Die Anzahl und Anordnung der Kameras wird dabei vorteilhaft anhand der Größe des Raums, in dem die Position der Patientenaufnahme ermittelt werden soll, und anhand der Genauigkeit, mit der die Position ermittelt werden soll, ausgewählt. Die mindestens zwei Kameras mit mindestens teilweise unterschiedlichen Sichtfeldern oder mit mindestens teilweise überlappenden Sichtfeldern können dabei zur Aufnahme mindestens eines ersten, an der Patientenaufnahme angeordneten Positionsreferenzelements, zur Aufnahme mindestens eines zweiten, an einem Kalibrierelement angeordneten Positionsreferenzelements und/oder zur Aufnahme mindestens eines dritten, an dem Bildgebungssystem angeordneten Positionsreferenzelements vorgesehen sein.

Ausführungsbeispiele der Erfindung werden im Folgenden anhand der Zeichnung erläutert. Es zeigen:
- Fig. 1 bis 4: schematische Darstellungen von Ausführungsbeispielen einer Patientenbestrahlungseinrichtung,
- Fig. 5: eine schematische Darstellung einer Patientenaufnahme in einer Ansicht von der dem Boden zugewandten Seite,
- Fig. 6 und 7: schematische Darstellungen einer Patientenaufnahme mit daran angeordneten unterschiedlichen Kalibrierelementen,
- Fig. 8 und 9: ein Ausführungsbeispiel einer Patientenaufnahme mit unterschiedlichen daran angeordneten Kalibrierelementen in schematischer Darstellung,
- Fig. 10: eine schematische Darstellung einer Patientenaufnahme bei der Ermittlung der Position einer Kamera eines Positionserfassungssystems,
- Fig. 11: eine perspektivische Darstellung eines Reflektors eines Lasertracking-systems,
- Fig. 12: den Reflektor aus Fig. 11 in Seitenansicht,
- Fig.13: eine schematische Darstellung einer Patientenbestrahlungseinrichtung mit einem an der Patientenaufnahme angeordneten Kalibrierelement,
- Fig. 14 und 15: schematische Darstellungen unterschiedlicher Lichtquellen des Positions-erfassungsystems,
- Fig. 16 und 17: Ausführungsbeispiele des Pösitionserfassungssystems in schematischer Darstellung,
- Fig. 18 bis 21: Ausführungsbeispiele für Positionsreferenzelemente,
- Fig. 22: ein Ablaufdiagramm eines Verfahrens zur Einrichtung einer Patientenbestrahlungseinrichtung,
- Fig. 23: ein Ablaufdiagramm eines Verfahrens zur Positionierung eines Patienten an einer Patientenbestrahlungseinrichtung.

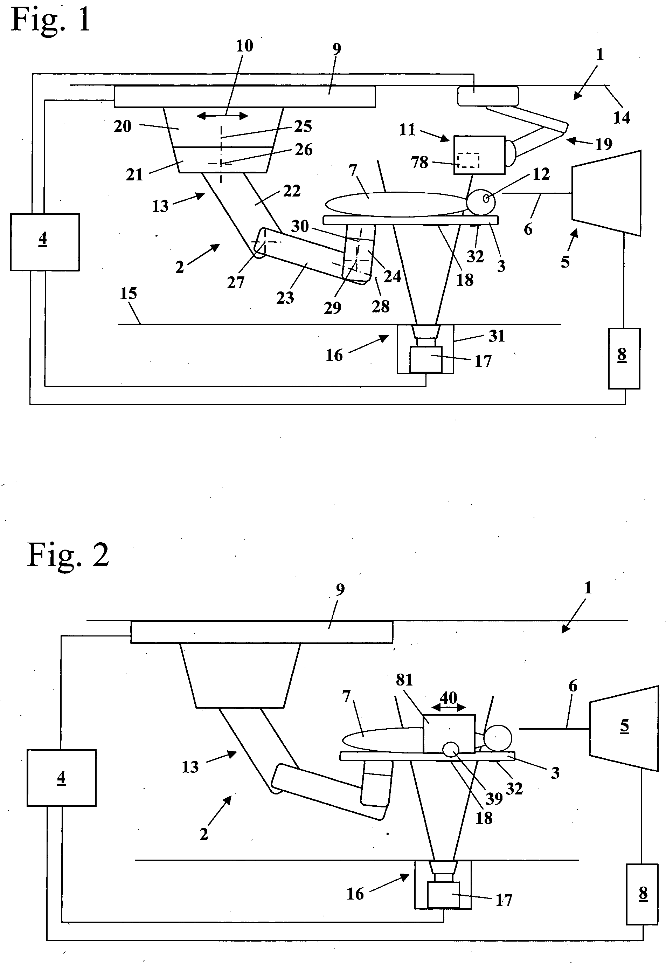

Fig. 1 zeigt schematisch ein Ausführungsbeispiel einer Patientenbestrahlungseinrichtung 1. Die Patientenbestrahlungseinrichtung 1 umfasst eine Patientenpositioniereinrichtung 2, mit der eine Patientenaufnahme 3 im Raum positionierbar ist. Die Patientenbestrahlungseinrichtung 1 umfasst eine Strahlenquelle 5, die einen schematisch dargestellten Strahl 6 zur Bestrahlung eines Patienten erzeugt. Die Strahlenquelle 5 kann beispielsweise eine Strahlenquelle für Protonen, Neutronen oder Ionen sein. Die Strahlenquelle 5 kann auch einen Photonenstrahl wie beispielsweise einen Röntgen- oder Gammastrahl erzeugen. Auch eine andere Art von Strahlung kann vorteilhaft sein. Die Patientenbestrahlungseinrichtung 1 kann zur Diagnose oder, besonders vorteilhaft, zur Therapie vorgesehen sein. Die Strahlenquelle 5 ist in den Figuren lediglich schematisch dargestellt und kann beispielsweise ein Zyklotron oder Synchrotron zur Strahlerzeugung umfassen. Die Strahlenquelle 5 wird über ein schematisch gezeigtes Steuerungssystem 8 angesteuert. Die Strahlenquelle 5 kann ortsfest im Raum angeordnet sein. Die Strahlenquelle 5 kann jedoch auch im Raum beweglich sein und vorzugsweise als Gantry ausgebildet sein, die um ein Isozentrum drehbar ist.

Die Patientenbestrahlungseinrichtung 1 besitzt ein Bildgebungssystem 11. Das Bildgebungssystem 11 ist in den Figuren lediglich schematisch dargestellt und umfasst vorteilhaft eine Strahlenquelle sowie einen Empfängerschirm. Das Bildgebungssystem 11 kann beispielsweise eine Röntgeneinheit, ein MRT oder dergleichen sein. Die Patientenbestrahlungseinrichtung 1 besitzt außerdem ein Positionserfassungssystem 16 zur Erfassung der Position der Patientenaufnahme 3. Die Patientenbestrahlungseinrichtung 1 besitzt eine Steuereinrichtung 4, die die Patientenpositioniereinrichtung 2, das Bildgebungssystem 11 und das Positionserfassungssystem 16 ansteuert. Die Steuereinrichtung 4 ist auch mit dem Steuerungssystem 8 der Strahlenquelle 5 verbunden. Das Steuerungssystem 8 kann auch in die Steuereinrichtung 4 integriert sein. Die Darstellung der Steuereinrichtung 4 ist lediglich schematisch. Die Steuereinrichtung 4 kann in mehrere Teile, beispielweise eine Steuereinrichtung zur Ansteuerung der Patientenpositioniereinrichtung 2, eine Steuereinrichtung zur Ansteuerung des Bildgebungssystems 11 sowie eine übergeordnete Steuereinrichtung zur Koordination der einzelnen Steuereinrichtungen geteilt sein.

Die Patientenpositioniereinrichtung 2 umfasst eine Linearachse 9, an der ein Roboterarm 13 gehalten ist. Im Ausführungsbeispiel ist die Linearachse 9 an einer Raumdecke 14 des Behandlungsraums angeordnet. Die Linearachse 9 kann jedoch auch an einem Raumboden 15 oder einer Vertiefung im Raumboden 15 angeordnet sein. Die Linearachse 9 kann auch entfallen, so dass der Roboterarm 13 unmittelbar an Raumdecke 14 oder Raumboden 15 festgelegt sein kann. Auch eine andere Anbringung des Roboterarms 13 kann vorteilhaft sein. Anstatt des Roboterarms 13 kann auch eine Patientenpositioniereinrichtung 2 vorgesehen sein, die mehrere Linearachsen umfasst, die translatorische Bewegungen zulassen, und die eine geringere Anzahl von Rotationsbewegungsachsen aufweist.

Die Linearachse 9 ermöglicht eine Bewegung des Roboterarms 13 gegenüber der Raumdecke 14 in Richtung eines Doppelpfeils 10. Der Roboterarm 13 führt gegenüber der Raumdecke 14 dabei eine translatorische Bewegung durch. Der Roboterarm 13 umfasst ein Grundgestell 20, an dem ein Karussell 21 um eine erste Rotationsbewegungsachse 25 drehbar gelagert ist. Die erste Rotationsbewegungsachse 25 ist im Ausführungsbeispiel senkrecht angeordnet. Am Karussell 21 ist eine Schwinge 22 um eine zweite Rotationsbewegungsachse 26 schwenkbar gehalten. Die zweite Rotationsbewegungsachse 26 ist im Ausführungsbeispiel horizontal angeordnet und verläuft senkrecht zur ersten Rotationsbewegungsachse 25. An dem dem Karussell 21 abgewandten Ende der Schwinge 22 ist ein Arm 23 um eine dritte Rotationsbewegungsachse 27 schwenkbar gehalten. Die dritte Rotationsbewegungsachse 27 ist parallel zur zweiten Rotationsbewegungsachse 26 und im Ausführungsbeispiel ebenfalls horizontal angeordnet. An dem der Schwinge 22 abgewandten Ende des Arms 23 ist eine Hand 24 vorgesehen, die Rotationen um eine vierte Rotationsbewegungsachse 28, eine fünfte Rotationsbewegungsachse 29 und eine sechste Rotationsbewegungsachse 30 zulässt. Die Rotationsbewegungsachsen 28, 29 und 30 sind so angeordnet, dass die Patientenaufnahme 3 gegenüber dem Arm 23 um alle drei Raumachsen verschwenkt werden kann.

Wie Fig. 1 zeigt, ist an der Patientenaufnahme 3 ein schematisch gezeigter Patient 7 angeordnet. Im Ausführungsbeispiel ist die Patientenaufnahme 3 eine Patientenliege, auf der der Patient 7 liegt. Die Patientenaufnahme 3 kann jedoch beispielsweise auch ein Stuhl oder dgl. sein. Der Patient 7 besitzt ein zu bestrahlendes Zielvolumen 12, beispielsweise einen zu bestrahlenden Tumor. Um im Betrieb den Strahl 6 möglichst genau auf das Zielvolumen 12 des Patienten 7 lenken zu können und umliegendes Gewebe nicht oder möglichst gering zu bestrahlen, ist das Positionserfassungssystem 16 vorgesehen. Über die Steuerung des Roboterarms 13 ist die ungefähre Lage der Patientenaufnahme 3 im Raum zwar bekannt, es hat sich jedoch gezeigt, dass eine höhere Genauigkeit bei der Positionierung der Patientenaufnahme 3 wünschenswert ist. Die Lage der Patientenaufnahme 3 kann dabei im Raumkoordinatensystem oder in einem Koordinatensystem, das einen Rückschluss auf die Lage im Raum zulässt, bekannt sein.

Im Ausführungsbeispiel besitzt das Positionserfassungssystem 16 mindestens eine Kamera 17, die in einer Vertiefung 31 am Boden angeordnet sein kann. Die Kamera 17 kann jedoch auch auf dem Raumboden 15, an einer Wand oder an der Raumdecke 14 angeordnet sein. An der Patientenaufnahme 3 ist mindestens ein erstes Positionsreferenzelement 18 angeordnet, das von der Kamera 17 aufgenommen wird. Über das erste Positionsreferenzelement 18 kann das Positionserfassungssystem 16 die Lage der Patientenaufnahme 3 ermitteln, wie im Folgenden noch näher erläutert wird. Das Positionserfassungssystem 16 ermittelt die Lage der Patientenaufnahme 3 vorteilhaft im raumeigenen Koordinatensystem. Auch eine Ermittlung in einem anderen Koordinatensystem kann unter Umständen vorteilhaft sein. Anstatt des Positionserfassungssystems 16 kann auch ein anderes Positionserfassungssystem vorgesehen sein, beispielsweise ein anderes optisches Positionserfassungssystem oder ein mechanisches Positionserfassungssystem. Ein mechanisches Positionserfassungssystem kann beispielsweise eine oder mehrere Koordinatenmesseinrichtungen wie beispielsweise einen Messarm umfassen und die Position der Patientenaufnahme 3 über eine mechanische Abtastung des mindestens einen ersten Positionsreferenzelements 18 erfassen. Auch ein anderes Positionserfassungssystem, beispielsweise ein Positionserfassungssystem, das mit Funkmarken oder dgl. arbeitet, kann vorteilhaft sein.

Das Bildgebungssystem 11 ist über eine Bewegungseinrichtung 19 im Raum beweglich gehalten. Das Bildgebungssystem 11 ist dabei gegenüber der Patientenaufnahme 3 und der Strahlenquelle 5 in mindestens einer Dimension bewegbar. In dem in Fig. 1 gezeigten Ausführungsbeispiel lässt die Bewegungseinrichtung 19, die nur schematisch gezeigt ist, translatorische Bewegungen in allen drei Raumrichtungen und rotatorische Bewegungen um alle drei Raumachsen zu. Über die Steuereinrichtung 4, die die Bewegungseinrichtung 19 ansteuert, wird dabei die Lage des Bildgebungssystems 11 erfasst. Die Lage des Bildgebungssystems 11 wird dabei vorteilhaft als relative Lage zu einer Ausgangslage über die Ansteuerung der Antriebe der Bewegungseinrichtung 19 ermittelt. Alternativ kann auch das Bildgebungssystem 11 mindestens ein drittes Positionsreferenzelement 78 aufweisen, das von dem Positionserfassungssystem 16 zur Ermittlung der Lage des Bildgebungssystems 11 ausgewertet werden kann und das in Fig. 1 mit gestrichelter Linie eingezeichnet ist.

Bei bekannten Bildgebungssystemen 11 kann aus einer vom Bildgebungssystem 11 erzeugten Aufnahme auf die Lage des aufgenommenen Objekts geschlossen werden. Dies kann beispielsweise über die Position des Objekts auf der Aufnahme und die Größe des Objekts auf der Aufnahme erfolgen. Die Lage des aufgenommenen Objekts wird dabei jedoch relativ zu der Strahlenquelle und dem Empfängerschirm ermittelt. Die Strahlenquelle und der Empfängerschirm sind in einem oder mehreren Gehäusen des Bildgebungssystems 11 gehalten. Die Positionierung von Strahlenquelle und Empfängerschirm ist dabei Toleranzen unterworfen. Es hat sich gezeigt, dass die Erfassung der Position des Bildgebungssystems 11 allein keine ausreichend hohe Genauigkeit liefert, um die Position des Zielvolumens 12 im Raum mit hoher Genauigkeit zu bestimmen. Um die genaue Position des Zielvolumens 12 im Raum ermitteln zu können, ist an der Patientenaufnahme 3 mindestens ein Bildgebungsreferenzelement 32, beispielsweise eine Röntgenmarke, angeordnet. Das Bildgebungsreferenzelement 32 ist ein Element, das vom Bildgebungssystem 11 abgebildet wird. Das erste Positionsreferenzelement 18 und das Bildgebungsreferenzelement 32 sind an der Patientenaufnahme 3 vorteilhaft in einem bekannten, festen Abstand zueinander angeordnet. Über die Position des ersten Positionsreferenzelements 18 kann mittels des Bildgebungsreferenzelements 32 und des bekannten Abstands zwischen erstem Positionsreferenzelement 18 und Bildgebungsreferenzelement 32 die Lage des Zielvolumens 12 genau ermittelt werden. Dabei wird vorteilhaft die Lage des Zielvolumens 12 im Raum ermittelt. Die Lage des Zielvolumens 12 kann jedoch auch in einem Koordinatensystem ermittelt werden, das einen Rückschluss auf die Lage im Raum zulässt.

In dem in Fig. 1 gezeigten Ausführungsbeispiel ist das Bildgebungsreferenzelement 32 fest an der Patientenaufnahme 3 gehalten. Es kann vorgesehen sein, die Position einer mit dem Bildgebungssystem 11 erzeugten Abbildung bei einer einmaligen oder einer in regelmäßigen Abständen zu wiederholenden Einrichtung der Patientenbestrahlungseinrichtung 1 über die erzeugte Abbildung des Bildgebungsreferenzelements 32, die relative Lage von Bildgebungsreferenzelement 32 und erstem Positionsreferenzelement 18 und die vom Positionserfassungssystem 16 ermittelte Lage des ersten Positionsreferenzelements 18 zu ermitteln. Da das Bildgebungsreferenzelement 32 fest an der Patientenaufnahme 3 gehalten ist, kann die Position der mit dem Bildgebungssystem 11 von dem Bildgebungsreferenzelement 32 erzeugten Abbildung jedoch auch während des Betriebs der Patientenbestrahlungseinrichtung 1, vorzugsweise unmittelbar vor der Bestrahlung eines Patienten 7, erneut ermittelt oder überprüft werden. Vorteilhaft wird dabei die Lage der Abbildung im Raum, also in einem raumeigenen Koordinatensystem, ermittelt. Die Bestimmung der Lage der Abbildung kann jedoch auch in einem anderen Koordinatensystem, beispielsweise einem Koordinatensystem des Bildgebungssystems 11, erfolgen. Das Bildgebungsreferenzelement 32 kann jedoch auch abnehmbar an der Patientenaufnahme 3 gehalten und nur zeitweise an der Patientenaufnahme 3 angeordnet sein.

Fig. 2 zeigt ein Ausführungsbeispiel einer Patientenbestrahlungseinrichtung 1, die ein Bildgebungssystem 81 umfasst. Gleiche Bezugszeichen kennzeichnen in allen Figuren einander entsprechende Elemente. Der Aufbau der Patientenpositioniereinrichtung 2 entspricht dem zur Fig. 1 beschriebenen Aufbau. Gleiches gilt für das Positionserfassungssystem 16 und die Strahlenquelle 5 sowie für die Steuereinrichtung 4 und das Steuerungssystem 8.

Das Bildgebungssystem 81 ist an der Patientenaufnahme 3 gehalten und über eine Bewegungseinrichtung 39 an der Patientenaufnahme 3 in Richtung eines Doppelpfeils 40 beweglich. Das Bildgebungssystem 81 ist dabei nur in Längsrichtung der Patientenaufnahme 3 beweglich. Zusätzlich kann es vorteilhaft sein, dass das Bildgebungssystem 81 um die Patientenaufnahme 3 drehbar ist, so dass der Patient 7 aus unterschiedlichen Winkeln abgebildet werden kann. An der Patientenaufnahme 3 sind das mindestens eine erste Positionsreferenzelement 18 und das mindestens eine Bildgebungsreferenzelement 32 angeordnet.

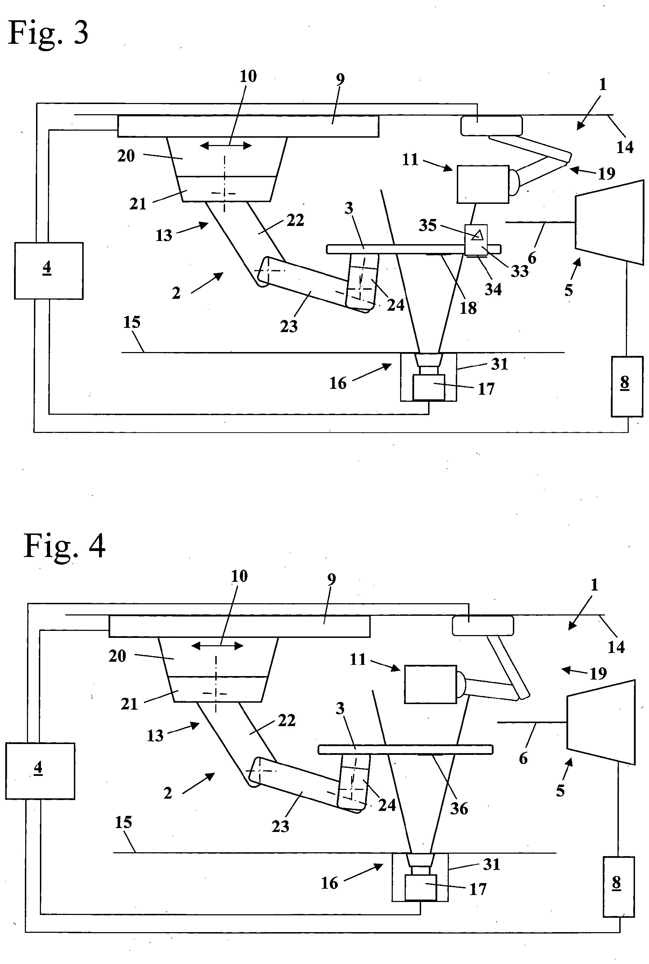

Fig. 3 zeigt ein Ausführungsbeispiel einer Patientenbestrahlungseinrichtung 1, bei der an der Patientenaufnahme 3 mindestens ein erstes Positionsreferenzelement 18 angeordnet ist. Zur Einrichtung der Patientenbestrahlungseinrichtung 1 wird an der Patientenaufnahme 3 ein Kalibrierelement 33 angeordnet, das mindestens ein zweites Positionsreferenzelement 34 sowie mindestens ein Bildgebungsreferenzelement 35 umfasst. Zur Ermittlung mindestens einer Information über die Position der mit dem Bildgebungssystem 11 von dem Bildgebungsreferenzelement 35 erzeugten Abbildung wird mit dem Bildgebungssystem 11 eine Aufnahme von dem Bildgebungsreferenzelement 35 gemacht. Aus dem Bild kann beispielsweise die Lage der Abbildung des Bildgebungsreferenzelements 35 relativ zum Bildgebungssystem 11 und, beispielsweise über die Größe der Abbildung, der Abstand zum Bildgebungssystem 11 ermittelt werden. Über die Abmessungen der Abbildung in unterschiedlichen Richtungen kann beispielsweise auf die Orientierung der Abbildung geschlossen werden.

In der gleichen Position von Bildgebungssystem 11 und Patientenaufnahme 3 wird mit der Kamera 17 die Position des ersten Positionsreferenzelements 18 ermittelt. Gleichzeitig wird die Position des am Kalibrierelement 33 angeordneten zweiten Positionsreferenzelements 34 ermittelt. Über den Abstand der Positionsreferenzelemente 18 und 34 und die bekannte Relativposition des Bildgebungsreferenzelements 35 zum zweiten Positionsreferenzelement 34 kann die Lage des Bildgebungsreferenzelements 35 ermittelt werden. Der Position des Bildgebungssystems 11, des ersten Positionsreferenzelements 18 und des Bildgebungsreferenzelements 35 wird mindestens eine Information zur Position der mit dem Bildgebungssystem 11 erzeugten Abbildung des Bildgebungsreferenzelements 35 zugeordnet. Die Position der Abbildung kann dabei beispielsweise in einem Koordinatensystem des Bildgebungssystems 11, also beispielsweise relativ zu einem Gehäuse des Bildgebungssystems 11, angegeben werden. Ausgehend von dieser einmal ermittelten Information zur Position der mit dem Bildgebungssystem erzeugten Abbildung kann über die von dem Bildgebungssystem 11 ausgehend von dieser Position ausgeführte Bewegung auf die Lage der mit dem Bildgebungssystem erzeugten Abbildung geschlossen werden. Dadurch ist eine sehr genaue Bestimmung der Lage eines Zielvolumens 12 eines Patienten 7 (Fig. 1) möglich. Über das Positionserfassungssystem 16 kann gleichzeitig die Position und Lage der Patientenaufnahme 3 genau bestimmt werden. Die Information über die Position und Lage der Patientenaufnahme 3 wird während des Betriebs insbesondere für eine schnelle Positionierung des Zielvolumens 12 an einer gewünschten Soll-Position, beispielsweise in der vorgesehenen Bestrahlungsposition, genutzt. Die Positionen können jeweils in einem raumfesten Koordinatensystem oder in einem anderen Koordinatensystem, das einen Rückschluss auf die Lage im Raumkoordinatensystem zulässt, ermittelt werden.

Fig. 4 zeigt ein weiteres Ausführungsbeispiel einer Patientenbestrahlungseinrichtung 1, bei der ein kombiniertes Referenzelement 36 an der Patientenaufnahme 3 gehalten ist. Der Aufbau der Patientenbestrahlungseinrichtung 1 entspricht im Wesentlichen dem zu Fig. 1 beschriebenen Aufbau. Das kombinierte Referenzelement 36 ist sowohl von dem Bildgebungssystem 11 abbildbar als auch von dem Positionserfassungssystem 16 erfassbar. Dadurch kann auf einfache Weise die Position der Patientenaufnahme 3 und gleichzeitig die Lage einer mit dem Bildgebungssystem 11 erzeugten Abbildung ermittelt werden.

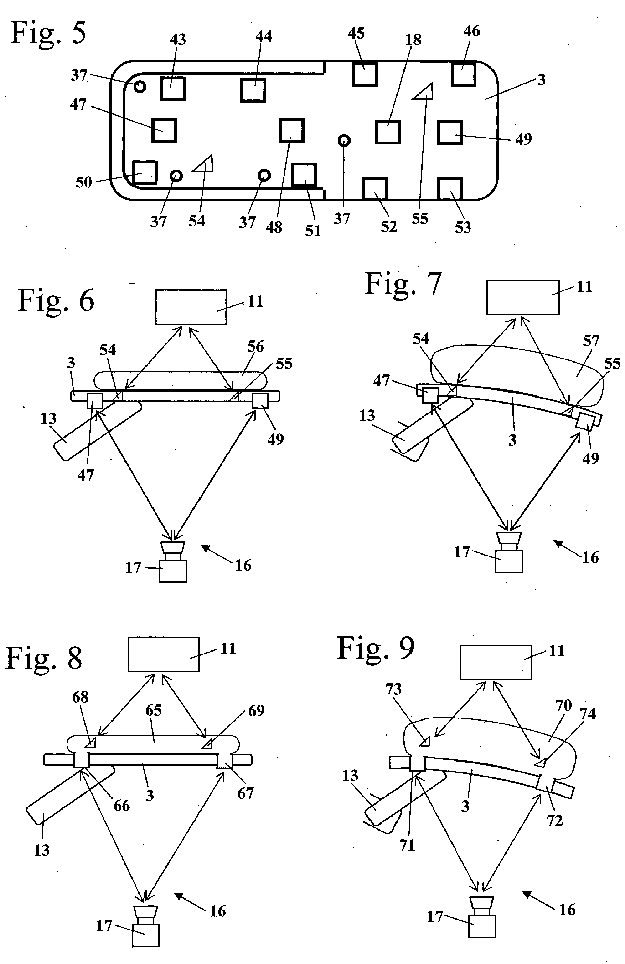

In den Figuren 1 bis 4 ist jeweils nur ein erstes Positionsreferenzelement 18 an der Patientenaufnahme 3 sowie ein Bildgebungsreferenzelement 32, 35 gezeigt. Um die Genauigkeit der Positionsbestimmung zu erhöhen, sind vorteilhaft eine Vielzahl von ersten Positionsreferenzelementen 18 und eine Vielzahl von Bildgebungsreferenzelementen 32, 35 vorgesehen. Dadurch kann die Lage der Patientenaufnahme 3 sehr genau ermittelt werden. Fig. 5 zeigt schematisch eine mögliche Anordnung von ersten Positionsreferenzelementen 18 und 43 bis 53 an der Unterseite einer Patientenaufnahme 3. Die ersten Positionsreferenzelemente 43 bis 53 sind über fast die gesamte Länge und die gesamte Breite der Patientenaufnahme 3 ungleichmäßig verteilt. Auch eine gleichmäßige Anordnung der ersten Positionsreferenzelemente 18 und 43 bis 53 kann jedoch vorteilhaft sein. Dadurch, dass die gesamte Breite und näherungsweise die gesamte Länge der Patientenaufnahme 3 ausgenutzt werden, kann eine hohe Genauigkeit erreicht werden. Das Positionserfassungssystem 16 kann dadurch ferner die Lage der Patientenaufnahme 3 auch dann noch ausreichend genau bestimmen, wenn sich nur einige der ersten Positions-referenzelemente 18 und 43 bis 53 im Sichtfeld der Kamera 17 des Positionserfassungssystems 16 befinden.

Im Ausführungsbeispiel sind an der Patientenaufnahme 3 zwei Bildgebungsreferenzelemente 54 und 55 vorgesehen. Es kann jedoch auch eine größere Anzahl von Bildgebungsreferenzelementen 54, 55 vorgesehen sein. Vorteilhaft sind die Bildgebungsreferenzelemente 54 und 55 so an der Patientenaufnahme 3 angeordnet, dass bei jeder Aufnahme eines Zielvolumens 12 mindestens ein Bildgebungsreferenzelement 54 oder 55 auf der Aufnahme sichtbar ist, das Bildgebungsreferenzelement 54, 55 jedoch keine Überschneidung mit dem Zielvolumen 12 aufweist und die abzubildenden Bereiche des Patienten 7 nicht überdeckt.

Im Ausführungsbeispiel sind sowohl die ersten Positionsreferenzelemente 18 und 43 bis 53 als auch die Bildgebungsreferenzelemente 54 und 55 so ausgebildet, dass sie nicht nur eine Information über die Lage der Patientenaufnahme 3 und der mit dem Bildgebungssystem 11, 81 erzeugten Abbildung, sondern auch über die Orientierung, also eine Kipp- oder Drehlage der Patientenaufnahme 3 bzw. der mit dem Bildgebungssystem 11, 81 erzeugten Abbildung liefern.

Je nach Gewicht eines an der Patientenaufnahme 3 angeordneten Patienten 7 kann sich eine mehr oder weniger starke Durchbiegung der Patientenaufnahme 3 ergeben. Dabei ändert sich die Lage des Zielvolumens 12 im Raum gegenüber einer nicht verformten Patientenaufnahme 3. Um den Einfluss des unterschiedlichen Gewichts von Patienten 7 mit zu berücksichtigen, ist vorgesehen, dass an der Patientenaufnahme 3 unterschiedliche Kalibrierelemente 56 und 57 angeordnet werden, wie in den Figuren 6 und 7 gezeigt ist. Im Ausführungsbeispiel ergibt sich bei dem in Fig. 6 gezeigten, ersten Kalibrierelement 56, das ein geringes Gewicht aufweist, im Wesentlichen keine Verformung der Patientenaufnahme 3. An der Patientenaufnahme 3 sind die beiden ersten Positionsreferenzelemente 47 und 49 und die beiden Bildgebungsreferenzelemente 54 und 55 angeordnet. Zur Ermittlung der Durchbiegung der Patientenaufnahme 3 werden bei an der Patientenaufnahme 3 angeordnetem erstem Kalibrierelement 56 die beiden Bildgebungsreferenzelemente 54 und 55 vom Bildgebungssystem 11 abgebildet. Alternativ oder zusätzlich kann die Lage der beiden ersten Positionsreferenzelemente 47 und 49 mit dem Positionserfassungssystem 16 erfasst werden. Über mindestens zwei Referenzelemente 54, 55 oder 47, 49 kann die Verformung der Patientenaufnahme 3 rechnerisch ermittelt werden.

Fig. 7 zeigt die Anordnung mit einem an der Patientenaufnahme 3 angeordneten zweiten Kalibrierelement 57, dessen Gewicht deutlich größer als das des Kalibrierelements 56 ist. Beim Kalibrierelement 57 ergibt sich eine deutliche Verformung der Patientenaufnahme 3. Auch bei an der Patientenaufnahme 3 angeordnetem zweiten Kalibrierelement 57 werden die Bildgebungsreferenzelemente 54 und 55 mit dem Bildgebungssystem 11 abgebildet und/oder die Lage der ersten Positionsreferenzelemente 47 und 49 wird mit dem Positionserfassungssystem 16 erfasst. Aufgrund des geänderten Abstands und der geänderten Raumlage der ersten Positiönsreferenzelemente 47 und 49 bzw. der Bildgebungsreferenzelemente 54 und 55 kann die Durchbiegung, also die Verformung der Patientenaufnahme 3, bei der Ermittlung der Position der mit dem Bildgebungssystem 11 erzeugten Abbildung mit berücksichtigt werden. Vorteilhaft werden eine Vielzahl von Kalibrierelementen 56 und 57 mit unterschiedlichem Gewicht an der Patientenaufnahme 3 angeordnet und die Durchbiegung ermittelt und einem dem jeweiligen Kalibrierelement entsprechenden Patientengewicht zugeordnet. Es kann vorteilhaft sein, mehr als zwei erste Positionsreferenzelemente 47, 49 bzw. mehr als zwei Bildgebungsreferenzelemente 54, 55 an den Kalibrierelementen 56, 57 vorzusehen.

Die Figuren 8 und 9 zeigen ein weiteres Ausführungsbeispiel zur Ermittlung der Verformung der Patientenaufnahme 3 bei unterschiedlichem Patientengewicht. In der Darstellung in Fig. 8 ist an der Patientenaufnahme 3 ein Kalibrierelement 65 angeordnet, das ein geringes Gewicht besitzt. Beim Kalibrierelement 65 ergibt sich im Wesentlichen keine Verformung der Patientenaufnahme 3. Das Kalibrierelement 65 besitzt im Ausführungsbeispiel zwei Bildgebungsreferenzelemente 68 und 69, die vom Bildgebungssystem 11 abgebildet werden. Alternativ oder zusätzlich können zwei zweite Positionsreferenzelemente 66, 67 an der Patientenaufnahme 3 vorgesehen sein, die vom Positionserfassungssystem 16 aufgenommen und deren Lage bestimmt werden kann.

In Fig. 9 ist die Patientenaufnahme 3 mit einem Kalibrierelement 70 gezeigt, dessen Gewicht deutlich größer als das des Kalibrierelements 65 ist. Beim Kalibrierelement 70 ergibt sich eine deutliche Verformung der Patientenaufnahme 3. Das Kalibrierelement 70 besitzt im Ausführungsbeispiel zwei Bildgebungsreferenzelemente 73 und 74, die vom Bildgebungssystem 11 abbildbar sind. Über die relative Lage der Abbildungen der Bildgebungsreferenzelemente 73 und 74 zueinander kann die Durchbiegung der Patientenaufnahme 3 ermittelt werden. Alternativ oder zusätzlich sind zwei zweite Positionsreferenzelemente 71 und 72 am Kalibrierelement 70 vorgesehen, die von der Kamera 17 erfasst und deren Lage ermittelt werden kann, um die Durchbiegung der Patientenaufnahme 3 zu ermitteln. Es kann auch vorteilhaft sein, die Durchbiegung der Patientenaufnahme 3 sowohl über das Bildgebungssystem 11 als auch über das Positionserfassungssystem 16 zu ermitteln, um eine höhere Genauigkeit zu erreichen. Auch eine größere Anzahl von Bildgebungsreferenzelementen 68, 69, 73, 74 und/oder von zweiten Positionsreferenzelementen 66, 67, 71, 72 kann vorteilhaft sein.

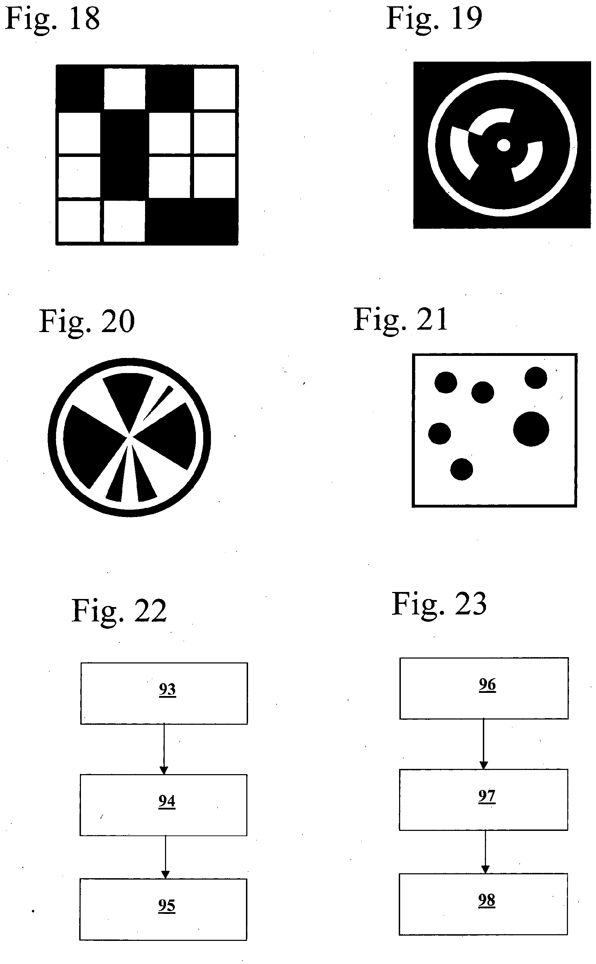

Ein einfacher Aufbau des Positionserfassungssystems 16 ergibt sich, wenn das Positionserfassungssystem 16 mindestens eine Kamera 17 umfasst und die Positionsreferenzelemente 18, 34 und 43 bis 53, 66, 67, 71, 72 und 78 fotogrammetrische Marken sind. Die Figuren 18 bis 21 zeigen unterschiedliche Ausführungsbeispiele solcher fotogrammetrischer Marken. Die fotogrammetrischen Marken lassen vorteilhaft sowohl einen Rückschluss auf die Lage über die Entfernung zur Kamera 17 und die Richtung, in der das Positionsreferenzelement 18, 34, 43 bis 53, 66, 67, 71, 72 und 78 gegenüber der Kamera 17 liegt, zu, als auch über die Verzerrung der Strukturen einen Rückschluss über die Orientierung, also die Drehlage um alle drei Raumachsen. Ist die Position und Orientierung der Kamera 17 im Raum bekannt, so kann über die Kamera 17 die Position der Positionsreferenzelemente 18, 34 und 43 bis 53, 66, 67, 71, 72 und 78 im Raum, also im raumeigenen Koordinatensystem, ermittelt werden.

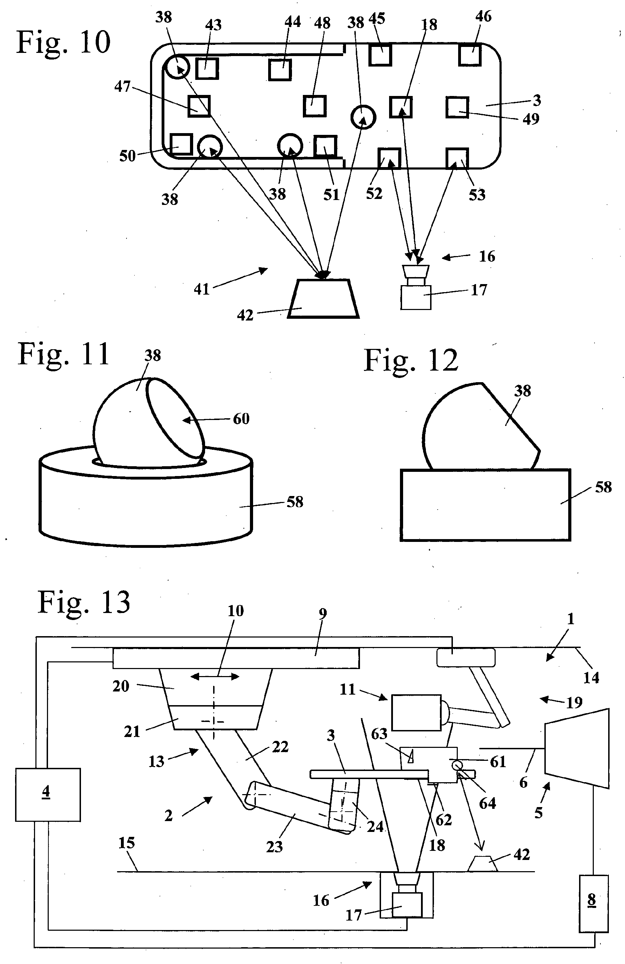

Die Kalibrierung des Positionserfassungssystems 16 erfolgt vorzugsweise mittels eines in Fig. 10 schematisch gezeigten Lasertrackingsystems 41. Wie Fig. 5 zeigt, besitzt die Patientenaufnahme 3 mehrere Aufnahmen 37. Die Aufnahmen 37 dienen zur Aufnahme von in den Figuren 11 und 12 gezeigten Reflektoren 38 des Lasertrackingsystems 41. Die Aufnahmen 37 sind im Ausführungsbeispiel Passpunkte für die Aufnahme von Haltern 58 der Reflektoren 38. Es kann auch vorgesehen sein, die Reflektoren 38 direkt an den Aufnahmen 37 anzubringen. Die Position der Aufnahmen 37 muss mit hoher Genauigkeit bekannt sein. Hierzu können die Aufnahmen 37 mit sehr hoher Genauigkeit an der Patientenaufnahme 3 angeordnet werden. Es kann auch vorgesehen sein, die Passpunkte mit geringer Genauigkeit an der Patientenaufnahme 3 anzuordnen und die Position der Passpunkte mit sehr hoher Genauigkeit zu bestimmen, beispielsweise mittels eines Messarms oder dgl..

Die an der Patientenaufnahme 3 angeordneten ersten Positionsreferenzelemente 18 und 43 bis 53 werden von der Kamera 17 in mindestens einer Lage der Patientenaufnahme 3 aufgenommen. Vorzugsweise werden in einer Vielzahl unterschiedlicher Richtungen von der Kamera 17 Aufnahmen gemacht. Dabei können die Kamera 17 oder die Patientenaufnahme 3 oder beide bewegt werden. Aus diesen Aufnahmen wird ein Referenzpunktefeld, nämlich eine definierte Punktewolke, erstellt. Dem Referenzpunktefeld wird ein Maßstab zugeordnet. Hierzu können in den Aufnahmen 37 nicht gezeigte Halbkugeln angeordnet werden, die einen fotogrammetrischen Punkt aufweisen. Um den Fehler bei der Kalibrierung zu minimieren, kann vorgesehen sein, die Halbkugeln mit fotogrammetrischem Punkt nicht unmittelbar in den Aufnahmen 37, sondern an Haltern 58 (Fig. 11) der Reflektoren 38 anzuordnen. Diese fotogrammetrischen Punkte können beispielsweise gemeinsam mit den ersten Positionsreferenzelementen 18 und 43 bis 53 von der Kamera abgebildet werden. Ist der Abstand der Aufnahmen 37 bekannt, so kann hieraus ein Maßstab des Referenzpunktefelds ermittelt werden. Alternativ kann der Abstand der Aufnahmen 37 beispielsweise mit dem Lasertrackingsystem 41, mit einem Messarm oder dgl. ermittelt werden.

Anschließend werden in den Aufnahmen 37 die Reflektoren 38 angeordnet. Jeder Reflektor 38 besitzt eine nicht im Einzelnen gezeigte Reflexionsspiegelanordnung 60. Die Reflektoren 38 sind vorteilhaft sphärisch montierte Retroreflektoren (SMR). Das Lasertrackingsystem 41 umfasst einen Lasertracker 42. Zunächst wird die Position des Lasertrackers 42 im Raum vermessen. Anschließend werden in mindestens einer Position der Patientenaufnahme 3 die ersten Positionsreferenzelemente 18 und 43 bis 53 von der Kamera 17 aufgenommen und die Lage der Patientenaufnahme 3 über die Reflektoren 38 mit dem Lasertracker 42 ermittelt. Über die Lage der Reflektoren 38 ist auch deren Abstand bekannt, der zur Ermittlung des Maßstabs des Referenzpunktefelds genutzt werden kann. Vorteilhaft wird in mehreren Positionen der Patientenaufnahme 3 sowohl die Position mindestens eines Reflektors 38 gemessen als auch die Anordnung mindestens eines ersten Positionsreferenzelements 18 und 43 bis 53 mit der Kamera 17 aufgenommen. Zur Ermittlung der Position der Patientenaufnahme 3 kann die Anordnung eines Reflektors 38 an der Patientenaufnahme 3 ausreichend sein. Um zusätzlich die Orientierung im Raum ermitteln zu können, sind vorteilhaft drei Reflektoren 38 vorgesehen, die an bekannten Positionen an der Patientenaufnahme 3 angeordnet sind.

Mit Hilfe des Referenzpunktefelds wird in jeder Position der Patientenaufnahme 3 die Position der Patientenaufnahme 3 im kameraeigenen Koordinatensystem über die Abbildung des mindestens einen ersten Positionsreferenzelements 18 und 43 bis 53 bestimmt. Gleichzeitig wird die genaue Position der Patientenaufnahme 3 im Raum über das Lasertrackingsystem 41 ermittelt. Über die Zuordnung der Werte wird die Position der Kamera 17 im Raumkoordinatensystem berechnet. Anschließend werden die Reflektoren 38 aus den Aufnahmen 37 entfernt. Im Betrieb genügt es, die Position der Patientenaufnahme 3 im Raum nur mit Hilfe der Kamera 17 zu bestimmen, da die Position der Kamera 17 im Raum unveränderlich und bekannt ist und die relative Lage der Patientenaufnahme 3 zur Kamera 17 über das von der Kamera 17 aufgenommene Bild der ersten Positionsreferenzelemente 18 und 43 bis 53 ermittelt werden kann. Durch geeignete Auswahl von ersten Positionsreferenzelementen 18 und 43 bis 53 sowie deren geeignete Anordnung kann eine sehr hohe Genauigkeit von besser 1 mm bei der Bestimmung der Position der Patientenaufnahme 3 erreicht werden. Auch die Anzahl von ersten Positionsreferenzelementen kann geeignet gewählt werden. Es kann auch ein einziges erstes Positionsreferenzelement ausreichend sein.

Fig. 13 zeigt ein alternatives Ausführungsbeispiel, bei dem über ein Kalibrierelement 61, das zeitweise an der Patientenaufnahme 3 angeordnet wird, sowohl die Position einer mit dem Bildgebungssystem 11 erzeugten Abbildung als auch die Position der Kamera 17 ermittelt wird. Das Kalibrierelement 61 besitzt mindestens ein zweites Positionsreferenzelement 62, das von der Kamera 17 erfassbar ist. Das Kalibrierelement 61 besitzt außerdem mindestens ein von dem Bildgebungssystem 11 abbildbares Bildgebungsreferenzelement 63 sowie mindestens einen Reflektor 64. Die Anordnung des zweiten Positionsreferenzelements 62, des Bildgebungsreferenzelements 63 und des Reflektors 64 an dem Kalibrierelement 61 ist bekannt. Beispielsweise kann die Anordnung durch entsprechende, mit sehr hoher Genauigkeit hergestellte Aufnahmen für die Referenzelemente an dem Kalibrierelement 61 bekannt sein. Alternativ können die Positionen von zweitem Positionsreferenzelement 62, Bildgebungsreferenzelement 63 und Reflektor 64 an dem Kalibrierelement 61 mit sehr hoher Genauigkeit ausgemessen werden.

Zur Einrichtung der Patientenbestrahlungseinrichtung 1 wird die Position des Reflektors 64 im Raum mittels eines Lasertrackers 42 ermittelt. Die Position kann dabei direkt gemessen oder indirekt aus einer Messung des Lasertrackers 42 ermittelt werden. Die Anordnung des mindestens einen zweiten Positionsreferenzelements 62 an dem Kalibrierelement 61 wird mit der mindestens einen Kamera 17 ermittelt und die Position des Bildgebungsreferenzelementes 63 mit dem Bildgebungssystem 11 erfasst. Da die Lage des Reflektors 64 mit dem Lasertracker 42 mit sehr hoher Genauigkeit ermittelt werden kann, kann auch die Lage der Abbildung des Bildgebungssystems 11 über die Abbildung des Bildgebungsreferenzelements 63 mit sehr hoher Genauigkeit bestimmt werden. Die Bestimmung der Position der Kamera 17 kann über das von der Kamera 17 aufgenommene Bild des zweiten Positionsreferenzelements 62 erfolgen. Dadurch können Aufnahmen 37 für Reflektoren 38 an der Patientenaufnahme 3 entfallen. Es kann ein Kalibrierelement 61 vorgesehen werden, das zur Kalibrierung einer Vielzahl von Patientenbestrahlungseinrichtungen 1 genutzt werden kann. Eine genaue Positionierung des Kalibrierelements 61 an der Patientenaufnahme 3 ist nicht notwendig, da die Position des Kalibrierelements 61 über den Reflektor 64 vom Lasertracker 42 mit hoher Genauigkeit ermittelt wird.

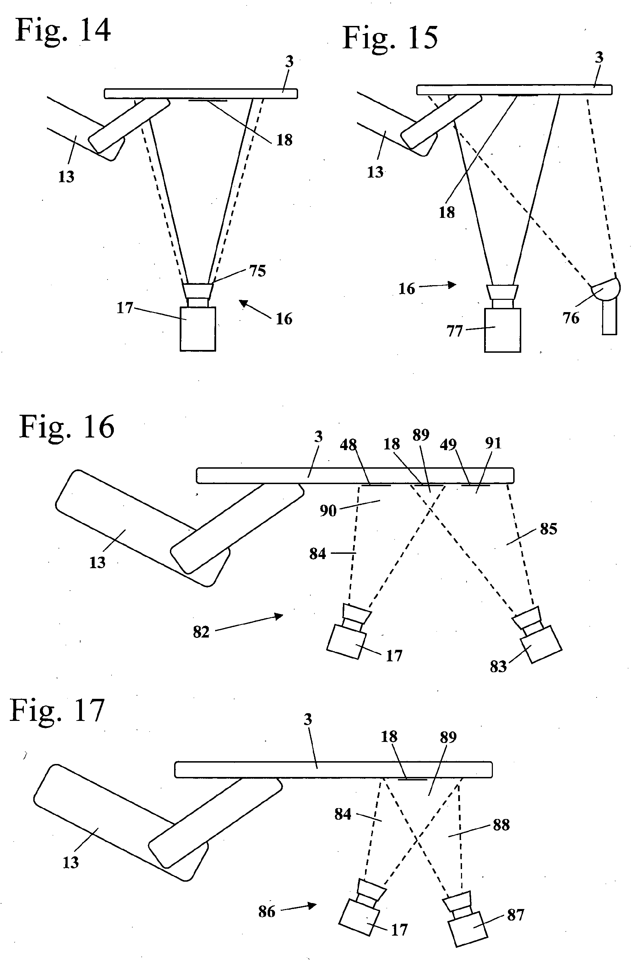

Es hat sich gezeigt, dass sich eine hohe Genauigkeit bei der Positionierung eines Patienten 7 an einer Patientenbestrahlungseinrichtung 1 erreichen lässt, wenn die ersten Positionsreferenzelemente 18 und 43 bis 53 gut angestrahlt werden. Beim Ausführungsbeispiel nach Fig. 14 ist an der Kamera 17 selbst eine Lichtquelle 75 integriert. Die Lichtquelle 75 ist im Ausführungsbeispiel als Lichtring um die Kamera 17 angeordnet. Beim Ausführungsbeispiel nach Fig. 15 ist eine Kamera 77 vorgesehen, die eine separate Lichtquelle 76 aufweist. Die Lichtquelle 75, 76 ist vorteilhaft eine Lichtquelle, die sichtbares Licht oder Infrarotlicht erzeugt. Als besonders vorteilhaft wird Infrarotlicht angesehen, da sichtbares Licht vom Patienten 7 als störend empfunden werden kann.

Fig. 16 zeigt ein Ausführungsbeispiel eines Positionserfassungssystems 82, das zwei Kameras 17, 83 umfasst. Wie Fig. 16 zeigt, überlappen sich die Sichtfelder 84 und 85 der Kameras 17 und 83 nur in einem kleinen ersten Raumbereich 89. In einem zweiten Raumbereich 90 werden erste Positionsreferenzelemente 48 nur von der Kamera 17 erfasst. In einem dritten Raumbereich 91 werden erste Positionsreferenzelemente 49 nur von der Kamera 83 erfasst. Lediglich in dem ersten Raumbereich 89, in dem sich die Sichtfelder 84 und 85 überschneiden, werden erste Positionsreferenzelemente 18 von beiden Kameras 17 und 83 erfasst. Dadurch, dass sich die Sichtfelder 84 und 85 nur teilweise überlappen, kann eine große Reichweite des Positionserfassungssystems 82 erreicht werden. Entsprechend können zweite, an einem Kalibrierelement 33, 61, 65, 70 angeordnete Positionsreferenzelemente 34, 62, 66, 67, 71, 72 oder dritte, an dem Bildgebungssystem 11, 81 angeordnete Positionsreferenzelemente 78 mit der Kamera 17 oder der Kamera 83 abgebildet werden.

Fig. 17 zeigt ein Ausführungsbeispiel eines Positionserfassungssystems 86, das eine zweite Kamera 87 aufweist. Die zweite Kamera 87 besitzt ein Sichtfeld 88, das sich in einem Raumbereich 89 mit dem Sichtfeld 84 der Kamera 17 überschneidet. Im Raumbereich 89 kann die Position der Patientenaufnahme 3 dadurch mit sehr hoher Genauigkeit bestimmt werden. Im Ausführungsbeispiel nach Fig. 17 überlappen sich die Sichtfelder 84 und 88 an der Patientenaufnahme 3 vollständig. Vorteilhaft ist die gezeigte Position der Patientenaufnahme 3 eine Position, in der bevorzugt eine Bestrahlung des Zielvolumens 12 stattfindet, so dass sich für die Positionierung des Ziel volumens 12 an der Strahlenquelle 5 eine sehr hohe Genauigkeit erreichen lässt. Entsprechend können zweite, an einem Kalibrierelement 33, 61, 65, 70 angeordnete Positionsreferenzelemente 34, 62, 66, 67, 71, 72 oder dritte, an dem Bildgebungssystem 11, 81 angeordnete Positionsreferenzelemente 78 sowohl mit der Kamera 17 als auch mit der Kamera 87 abgebildet werden, um eine hohe Genauigkeit zu erreichen.

Fig. 22 zeigt schematisch ein Ablaufdiagramm eines Verfahrens zur Einrichtung einer Patientenbestrahlungseinrichtung 1. In einem ersten Verfahrensschritt 93 wird das Positionserfassungssystem 16 kalibriert. Dies erfolgt vorteilhaft mittels eines Lasertrackingsystems 41, wie zu den Figuren 10 bis 12 oder zur Fig. 13 beschrieben.

In einem zweiten Verfahrensschritt 94 wird mindestens ein Bildgebungsreferenzelement 32, 35, 54, 55, 63, 68, 69, 73, 74 mit dem Bildgebungssystem 11, 81 abgebildet und mindestens eine Information über die Position der mit dem Bildgebungssystem 11, 81 erzeugten Abbildung mindestens eines Bildgebungsreferenzelements 32, 35, 54, 55, 63, 68, 69, 73, 74 ermittelt. Die Position des Bildgebungssystems 11, 81 kann über die Mittel zur Ermittlung einer Position des Bildgebungssystems 11, 81, beispielsweise einen Antrieb des Bildgebungssystems 11, 81 oder mindestens ein an dem Bildgebungssystem 11, 81 angeordnetes drittes Positionsreferenzelement 78, ermittelt werden. Dabei kann die absolute Position des Bildgebungssystems 11, 81 oder eine Relativposition bezogen auf eine bekannte Ausgangsposition ermittelt werden. Die Position des mindestens einen ersten Positionsreferenzelements 18, 43 bis 53 wird über das Positionserfassungssystem 16, 82, 86 ermittelt, und dieser Position des Bildgebungssystems 11, 81, des Bildgebungsreferenzelements 32, 35, 54, 55, 63, 68, 69, 73, 74 und des mindestens einen ersten Positionsreferenzelements 18, 43 bis 53 wird die Information über die Position der Abbildung zugeordnet.

In einem weiteren Verfahrensschritt 95, der vor oder nach dem Verfahrensschritt 94 stattfinden kann, werden an der Patientenaufnahme 3 mindestens zwei Kalibrierelemente 56, 57, 65, 70 mit unterschiedlichem Gewicht angeordnet, wie zu den Figuren 6 bis 9 beschrieben, und mit dem Positionserfassungssystem 16, 82, 86 Positionsreferenzelemente 47, 49, 66, 67, 71, 72 oder mit dem Bildgebungssystem 11, 81 Bildgebungsreferenzelemente 54, 55, 68, 69, 73, 74 abgebildet und deren relative Lage zueinander einem dem Gewicht des Kalibrierelements 56, 57, 65, 70 entsprechenden Patientengewicht zugeordnet.

Fig. 23 zeigt schematisch den Ablauf eines Verfahrens zur Positionierung eines Patienten 7 an einer Patientenbestrahlungseinrichtung 1. In einem ersten Verfahrensschritt 96 wird die Position mindestens eines an der Patientenaufnahme 3 angeordneten ersten Positionsreferenzelements 18, 43 bis 53 mit einem Positionserfassungssystem 16, 82, 86 ermittelt. In einem zweiten Verfahrensschritt 97 wird das Zielvolumen 12 mit dem Bildgebungssystem 11, 81 abgebildet und die Abweichung der tatsächlichen Position des Zielvolumens 12 zu einer Soll-Position des Zielvolumens 12 eines Patienten 7 anhand mindestens einer Information zur Position einer mit einem Bildgebungssystem 11, 81 erzeugten Abbildung ermittelt. Im Ausführungsbeispiel ist der zweite Verfahrensschritt 97 nach dem ersten Verfahrensschritt 96 vorgesehen. Es kann jedoch auch vorgesehen sein, dass der zweite Verfahrensschritt 97 vor dem ersten Verfahrensschritt 96 stattfindet. In einem nachfolgenden Verfahrensschritt 98 wird die Position des Zielvolumens 12 durch entsprechende Bewegung der Patientenaufnahme 3 mit der Patientenpositioniereinrichtung 2 korrigiert, bis sich das Zielvolumen 12 in seiner Soll-Position befindet.

Bei bekannten Patientenbestrahlungseinrichtungen ist zwar die Stellung des Bildgebungssystems z. B. über die Antriebe bekannt, die Genauigkeit kann jedoch für eine genaue Positionsbestimmung unzureichend sein. Vorteilhaft bildet das Bildgebungssystem neben dem Zielvolumen deshalb immer auch mindestens ein Bildgebungsreferenzelement ab, das fest und in bekanntem Abstand zu dem mindestens einen ersten Positionsreferenzelement an der Patientenaufnahme angebracht ist. Stattdessen kann auch vorgesehen sein, dass die Bestimmung der Position des Bildgebungssystems über die Antriebe und die Berücksichtigung der tatsächlichen Position der bildgebenden Einrichtungen erfolgt.

## Patentansprüche

1. Verfahren zur Einrichtung einer Patientenbestrahlungseinrichtung, wobei die Patientenbestrahlungseinrichtung (1) eine Patientenaufnahme (3), eine Patientenpositioniereinrichtung (2), an der die Patientenaufnahme (3) im Raum bewegbar gehalten ist, mindestens eine Strahlenquelle (5) zur Bestrahlung eines Zielvolumens (12) eines Patienten (7) und ein Bildgebungssystem (11, 81) zur Abbildung des Zielvolumens (12) umfasst, wobei die Patientenbestrahlungseinrichtung (1) mindestens ein Positionserfassungssystem (16, 82, 86) zur Erfassung mindestens einer Information über die Position der Patientenaufnahme (3) im Raum umfasst, wobei das Positionserfassungssystem (16, 82, 86) mindestens ein an der Patientenaufnahme (3) angeordnetes erstes Positionsreferenzelement (18, 43 - 53) umfasst, und wobei an der Patientenaufnahme (3) mindestens zeitweise mindestens ein von dem Bildgebungssystem (11, 81) abbildbares Bildgebungsreferenzelement (32, 35, 54, 55, 63, 68, 69, 73, 74) gehalten ist,
wobei das Bildgebungssystem (11, 81) in mindestens einer Dimension gegenüber der Strahlenquelle (5) und der Patientenaufnahme (3) im Raum bewegbar ist, wobei das Bildgebungssystem (11, 81) Mittel zur Erfassung mindestens einer Information über die Position des Bildgebungssystems (11, 81) aufweist, wobei zur Einrichtung der Patientenbestrahlungseinrichtung (1) in mindestens einer Position von Patientenaufnahme (3) und Bildgebungssystem (11, 81) das Bildgebungsreferenzelement (32, 35, 54, 55, 63, 68, 69, 73, 74) mit dem Bildgebungssystem (11, 81) abgebildet und die Position des Bildgebungsreferenzelements (32, 35, 54, 55, 63, 68, 69, 73, 74) ermittelt wird, wobei bei der Einrichtung der Patientenbestrahlungseinrichtung (1) in der mindestens einen Position von Patientenaufnahme (3) und Bildgebungssystem (11, 81), in der das Bildgebungsreferenzelement (32, 35, 54, 55, 63, 68, 69, 73, 74) mit dem Bildgebungssystem (11, 81) abgebildet und die Position des Bildgebungsreferenzelements (32, 35, 54, 55, 63, 68, 69, 73, 74) ermittelt wird,
- die Position des mindestens einen ersten Positionsreferenzelements (18, 43 - 53) von dem Positionserfassungssystem (16, 82, 86) ermittelt wird und
- dieser Position des Bildgebungssystems (11, 81), des Bildgebungsreferenzelements (32, 35, 54, 55, 63, 68, 89, 73, 74) und des ersten Positionsreferenzelements (18, 43 - 53) mindestens eine Information zur Position der mit dem Bildgebungssystem (11, 81) von dem Bildgebungsreferenzelement (32, 35, 54, 55, 63, 68, 69, 73, 74) erzeugten Abbildung zugeordnet wird.

2. Verfahren nach Anspruch 1,
**dadurch gekennzeichnet, dass** die Position des mindestens einen Bildgebungsreferenzelements (32, 54, 55) anhand der relativen Lage des mindestens einen Bildgebungsreferenzelements (32, 54, 55) zu dem mindestens einen ersten Positionsreferenzelement (18, 43 - 53) ermittelt wird.

3. Verfahren nach Anspruch 1,
**dadurch gekennzeichnet, dass** mindestens ein zweites Positionsreferenzelement (34, 62) in bekannter relativer Lage zu dem mindestens einen Bildgebungsreferenzelement (35, 63) vorgesehen ist und dass die Position des mindestens einen Bildgebungsreferenzelements (35, 63) über eine vom Positionserfassungssystem (16, 82, 86) ermittelte Position des mindestens einen zweiten Positionsreferenzelements (34, 62) ermittelt wird.

4. Verfahren nach Anspruch 1,
**dadurch gekennzeichnet, dass** das mindestens eine erste Positionsreferenzelement und das mindestens eine Bildgebungsreferenzelement als mindestens ein von dem Bildgebungssystem abbildbares und von dem Positionserfassungssystem erfassbares, kombiniertes Referenzelement (36) ausgebildet sind und die Position des mindestens einen kombinierten Referenzelements (36) über das Positionserfassungssystem (16, 82, 86) ermittelt wird.

5. Verfahren nach einem der Ansprüche 1 bis 4,
**dadurch gekennzeichnet, dass** das Positionserfassungssystem (16, 82, 86) mindestens eine Information über die Orientierung des mindestens einen Positionsreferenzelements (18, 34, 43 - 53, 62, 66, 67, 71, 72, 78) im Raum ermittelt.

6. Verfahren nach einem der Ansprüche 1 bis 5,
**dadurch gekennzeichnet, dass** das Bildgebungssystem (11, 81) mindestens eine Information über die Orientierung des mindestens einen Bildgebungsreferenzelements (32, 35, 54, 55, 63, 68, 69, 73, 74) relativ zu dem Bildgebungssystem (11, 81) ermittelt.

7. Verfahren nach einem der Ansprüche 1 bis 6,
**dadurch gekennzeichnet, dass** das Positionserfassungssystem (16, 82, 86) mindestens eine Kamera (17, 77, 83, 87) umfasst und dass die Positionsreferenzelemente (18, 34, 43 - 53, 62, 66, 67, 71, 72, 78) fotogrammetrische Marken sind.

8. Verfahren nach Anspruch 7,
**dadurch gekennzeichnet, dass** die Kalibrierung des Positionserfassungssystems (16, 82, 86) mittels eines Lasertrackingsystems (41) erfolgt.

9. Verfahren nach Anspruch 8,
**dadurch gekennzeichnet, dass** die Patientenaufnahme (3) mindestens eine Aufnahme (37) für einen Reflektor (38) des Lasertrackingsystems (41) besitzt, wobei zur Ermittlung der Position der mindestens einen Kamera (17, 77, 83, 87) ein Reflektor (38) in der Aufnahme (37) angeordnet wird und in mindestens einer Position der Patientenaufnahme (3) sowohl die Position des Reflektors (38) mittels eines Lasertrackers (42) gemessen als auch die Anordnung des mindestens einen ersten Positionsreferenzelements (18, 43 - 53) mit der mindestens einen Kamera (17, 77, 83, 87) aufgenommen wird.

10. Verfahren nach Anspruch 8 oder 9,
**dadurch gekennzeichnet, dass** zur Einrichtung der Patientenbestrahlungseinrichtung (1) ein Kalibrierelement (61) an der Patientenaufnahme (3) angeordnet wird, wobei das Kalibrierelement (61) mindestens ein zweites Positionsreferenzelement (62), das mindestens eine von dem Bildgebungssystem (11, 81) erfassbare Bildgebungsreferenzelement (63) und mindestens einen Reflektor (64) des Lasertrackingsystems (41) aufweist, wobei die Anordnung des zweiten Positionsreferenzelements (62), des Bildgebungsreferenzelements (63) und des Reflektors (64) an dem Kalibrierelement (61) bekannt ist und dass die Position des Reflektors (64) im Raum mittels eines Lasertrackers (42) ermittelt, die Anordnung des mindestens einen zweiten Positionsreferenzelements (62) mit der mindestens einen Kamera (17, 77, 83, 87) aufgenommen und die Position des Bildgebungsreferenzelements (63) mit dem Bildgebungssystem (11, 81) erfasst wird und dass hieraus die Position des mindestens einen zweiten Positionsreferenzelements (62), mindestens eine Information über die Position der mit dem Bildgebungssystem (11, 81) von dem Bildgebungsreferenzelement (63) erzeugten Abbildung und die Position der Kamera (17, 77, 83, 87) ermittelt wird.

11. Verfahren nach einem der Ansprüche 7 bis 10,
**dadurch gekennzeichnet, dass** die Ermittlung der Position der Patientenaufnahme (3) über ein Referenzpunktefeld erfolgt, das aus mindestens einem von der mindestens einen Kamera (17, 77, 83, 87) aufgenommenen Bild des mindestens einen Positionsreferenzelements (18, 35, 43 - 53, 62) erzeugt wird.

12. Verfahren nach einem der Ansprüche 7 bis 11,
**dadurch gekennzeichnet, dass** zur Einrichtung der Patientenbestrahlungseinrichtung (1) nacheinander mindestens zwei Kalibrierelemente (56, 57, 65, 70) mit unterschiedlichem Gewicht an der Patientenaufnahme (3) angeordnet werden, wobei für jedes Kalibrierelement (56, 57, 65, 70) die relative Lage von mindestens zwei Referenzelementen (47, 49, 54, 55, 66 - 69, 71 - 74) zueinander ermittelt und einem dem Gewicht des Kalibrierelements (56, 57, 65, 70) entsprechenden Patientengewicht zugeordnet wird, wobei die mindestens zwei Referenzelemente (47, 49, 54, 55, 66 - 69, 71 - 74) insbesondere mindestens zwei Positionsreferenzelemente (47, 49, 66, 67, 71, 72) oder mindestens zwei Bildgebungsreferenzelemente (54, 55, 68, 69, 73, 74) sind.

13. Verfahren nach Anspruch 12,
**dadurch gekennzeichnet, dass** die mindestens zwei Referenzelemente (66, 67, 68, 69, 71, 72, 73, 74) an dem Kalibierelement (65, 70) angeordnet sind.

14. Verfahren nach Anspruch 12,
**dadurch gekennzeichnet, dass** die mindestens zwei Referenzelemente (47, 49, 54, 55) an der Patientenaufnahme (3) angeordnet sind.

15. Verfahren nach einem der Ansprüche 7 bis 14,
**dadurch gekennzeichnet, dass** das Positionserfassungssystem (16, 82, 86) mindestens eine Lichtquelle (75, 76) umfasst, die das mindestens eine Positionsreferenzelement (18, 34, 43 - 53, 62, 66, 67, 71, 72, 78) zumindest während einer Aufnahme anstrahlt.

16. Verfahren nach Anspruch 15,
**dadurch gekennzeichnet, dass** die Lichtquelle (75, 76) sichtbares Licht oder Infrarotlicht erzeugt.

17. Verfahren nach einem der Ansprüche 1 bis 16,
**dadurch gekennzeichnet, dass** das Bildgebungssystem (81) an der Patientenaufnahme (3) beweglich gehalten ist.

18. Verfahren nach einem der Ansprüche 1 bis 16,
**dadurch gekennzeichnet, dass** das Bildgebungssystem (11) unabhängig von der Patientenaufnahme (3) im Raum beweglich ist.

## Claims

1. Method for setting up a patient irradiation device, wherein the patient irradiation device (1) comprises a patent receptacle (3), a patient positioning device (2), on which the patent receptacle (3) is held movably in space, at least one radiation source (5) for irradiating a target volume (12) of a patient (7) and an imaging system (11, 81) for showing the target volume (12), wherein the patient irradiation device (1) comprises at least one position detecting system (16, 82, 86) for detecting at least one information on the position of the patent receptacle (3) in space, wherein the position detecting system (16, 82, 86) comprises at least one first position reference element (18, 43 - 53) located on the patent receptacle (3), and wherein at least temporarily at least one imaging reference element (32, 35, 54, 55, 63, 68, 69, 73, 74), which can be imaged by the imaging system (11, 81), is held on the patent receptacle (3),
wherein the imaging system (11, 81) is movable in space in at least one dimension relative to the radiation source (5) and the patent receptacle (3), wherein the imaging system (11, 81) has means for detecting at least one information on the position of the imaging system (11, 81), wherein, for setting up the patient irradiation device (1) in at least one position of patent receptacle (3) and imaging system (11, 81), the imaging reference element (32, 35, 54, 55, 63, 68, 69, 73, 74) is imaged with the imaging system (11, 81) and the position of the imaging reference element (32, 35, 54, 55, 63, 68, 69, 73, 74) is determined,
wherein, in the process of setting up the patient irradiation device (1) in the at least one position of patent receptacle (3) and imaging system (11, 81), in which the imaging reference element (32, 35, 54, 55, 63, 68, 69, 73, 74) is imaged with the imaging system (11, 81) and the position of the imaging reference element (32, 35, 54, 55, 63, 68, 69, 73, 74) is determined,
- the position of the at least one first position reference element (18, 43 - 53) is determined by the position detecting system (16, 82, 86) and
- at least one information on the position of the image generated with the imaging system (11, 81) by the imaging reference element (32, 35, 54, 55, 63, 68, 69, 73, 74) is assigned to this position of the imaging system (11, 81), the imaging reference element (32, 35, 54, 55, 63, 68, 69, 73, 74) and the first position reference element (18, 43 - 53).

2. Method according to claim 1,
**characterised in that** the position of the at least one imaging reference element (32, 54, 55) is determined from the position of the at least one imaging reference element (32, 54, 55) relative to the at least one the first position reference element (18, 43 - 53).

3. Method according to claim 1,
**characterised in that** at least one second position reference system (34, 62) is provided in a known position relative to the at least one imaging reference element (35, 63), and **in that** the position of the at least one imaging reference element (35, 63) is determined by way of a position of the at least one second position reference element (34, 62) determined by the position detecting system (16, 82, 86).

4. Method according to claim 1,
**characterised in that** the at least one first position reference element and the at least one imaging reference element are designed as at least one combined reference element (36), which can be imaged by the imaging system and detected by the position detecting system, and **in that** the position of the at least one combined reference element (36) is determined via the position detecting system (16, 82, 86).

5. Method according to any of claims 1 to 4,
**characterised in that** the position detecting system (16, 82, 86) determines at least one information on the orientation of the at least one position reference element (18, 34, 43 - 53, 62, 66, 67, 71, 72, 78) in space.

6. Method according to any of claims 1 to 5,
**characterised in that** the imaging system (11, 82) determines at least one information on the orientation of the at least one imaging reference element (32, 35, 54, 55, 63, 68, 69, 73, 74) relative to the imaging system (11, 82).

7. Method according to any of claims 1 to 6,
**characterised in that** the position detecting system (16, 82, 86) comprises at least one camera (17, 77, 83, 78), and **in that** the position reference elements (18, 43 - 53, 62, 66, 67, 71, 72, 78) are photogrammetric marks.

8. Method according to claim 7,
**characterised in that** the position detecting system (16, 82, 86) is calibrated by means of a laser tracking system (41).

9. Method according to claim 8,
**characterised in that** the patient receptacle (3) has at least one receptacle (37) for a reflector (38) of the laser tracking system (41), wherein, for determining the position of the at least one camera (17, 77, 83, 78), a reflector (38) is arranged in the receptacle (37), and in at least one position of the patient receptacle (3) both the position of the reflector (38) is measured by means of a laser tracker (42) and the arrangement of the at least one first position reference element (18, 43 - 53) is recorded with the at least one camera (17, 77, 83, 78).

10. Method according to claim 8 or 9,
**characterised in that**, for setting up the patient irradiation device (1), a calibrating element (61) is located on the patient receptacle (3), wherein the calibrating element (61) comprises at least one second position reference element (62), the at least one imaging reference element (63) detectable by the imaging system (11, 81) and at least one reflector (64) of the laser tracking system (41), wherein the arrangement of the at least one second position reference element (62), the imaging reference element (63) and the reflector (64) on the calibrating element (61) is known, and **in that** the position of the reflector (64) in space is determined by means of a laser tracker (42), the arrangement of the at least one second position reference element (62) is recorded with the at least one camera (17, 77, 83, 78) and the position of the imaging reference element (63) is detected with the imaging system (11, 81), and **in that** therefrom the position of the at least one second position reference element (62), at least one information on the position of the image generated with the imaging system (11, 81) by the imaging reference element (63) and the position of the camera (17, 77, 83, 78) are determined.

11. Method according to any of claims 7 to 10,
**characterised in that** the position of the patient receptacle (3) is determined by way of a reference point field generated from at least one image of the at least one position reference element (18, 35, 43 - 53, 62) recorded by the at least one camera (17, 77, 83, 78).

12. Method according to any of claims 7 to 11,
**characterised in that**, for setting up the patient irradiation device (1), at least two calibrating elements (56, 57, 65, 70) of different weight are consecutively arranged on the patient receptacle (3), wherein for each calibrating element (56, 57, 65, 70) the relative position of at least two reference elements (47, 49, 54, 55, 66 - 69, 71 - 74) is determined and a patient weight corresponding to the weight of the calibrating element (56, 57, 65, 70) is assigned, wherein the at least two reference elements (47, 49, 54, 55, 66 - 69, 71 - 74) are in particular st least two position reference elements (47, 49, 66, 67, 71, 72) or at least two imaging reference elements (54, 55, 68, 69, 73, 74).

13. Method according to claim 12,
**characterised in that** the at least two reference elements (66, 67, 68, 69, 71, 72, 73, 74) are located on the calibrating element (65, 70).

14. Method according to claim 12,
**characterised in that** the at least two reference elements (47, 49, 54, 55) are located on the patient receptacle (3).

15. Method according to any of claims 7 to 14,
**characterised in that** the position detecting system (16, 82, 86) comprises at least one light source (75, 76), which irradiates the at least one position reference system (18, 34, 43 - 53, 62, 66, 67, 71, 72, 78) at least during a exposure.

16. Method according to claim 15,
**characterised in that** the light source (75, 76) is visible light or infrared light.

17. Method according to any of claims 1 to 16,
**characterised in that** the imaging system (81) is movably held on the patient receptacle (3).

18. Method according to any of claims 1 to 16,
**characterised in that** the imaging system (11) is movable in space independently of the patient receptacle (3).

## Revendications

1. Procédé de montage d'un dispositif d'irradiation de patient, dans lequel le dispositif d'irradiation de patient (1) comprend un logement de patient (3), un dispositif de positionnement de patient (2), en lequel le logement de patient (3) est maintenu mobile dans l'espace, au moins une source de rayonnement (5) pour l'irradiation d'un volume cible (12) d'un patient (7) et un système d'imagerie (11, 81) pour la représentation du volume cible (12), dans lequel le dispositif d'irradiation de patient (1) comprend au moins un système de détection de position (16, 82, 86) pour la détection d'au moins une information sur la position du logement de patient (3) dans l'espace, dans lequel le système de détection de position (16, 82, 86) comprend au moins un premier élément de référence de position (18, 43 - 53) agencé en le logement de patient (3), et dans lequel au moins un élément de référence d'imagerie (32, 35, 54, 55, 63, 68, 69, 73, 74) pouvant être représenté par le système d'imagerie (11, 81) est maintenu au moins temporairement en le logement de patient (3),
dans lequel le système d'imagerie (11, 81) est mobile dans l'espace dans au moins une dimension par rapport à la source de rayonnement (5) et au logement de patient (3), dans lequel le système d'imagerie (11, 81) présente des moyens de détection d'au moins une information sur la position du système d'imagerie (11, 81), dans lequel pour le montage du dispositif d'irradiation de patient (1) dans au moins une position du logement de patient (3) et du système d'imagerie (11, 81), l'élément de référence d'imagerie (32, 35, 54, 55, 63, 68, 69, 73, 74) est représenté avec le système d'imagerie (11, 81) et la position de l'élément de référence d'imagerie (32, 35, 54, 55, 63, 68, 69, 73, 74) est déterminée, dans lequel lors du montage du dispositif d'irradiation de patient (1) dans l'au moins une position du logement de patient (3) et du système d'imagerie (11, 81), dans lequel l'élément de référence d'imagerie (32, 35, 54, 55, 63, 68, 69, 73, 74) est représenté avec le système d'imagerie (11, 81) et la position de l'élément de référence d'imagerie (32, 35, 54, 55, 63, 68, 69, 73, 74) est déterminée,
- la position de l'au moins un premier élément de référence de position (18, 43 - 53) est déterminée par le système de détection de position (16, 82, 86) et
- au moins une information sur la position de la représentation générée avec le système d'imagerie (11, 81) de l'élément de référence d'imagerie (32, 35, 54, 55, 63, 68, 89, 73, 74) est associée à cette position du système d'imagerie (11, 81), de l'élément de référence d'imagerie (32, 35, 54, 55, 63, 68, 69, 73, 74) et du premier élément de référence de position (18, 43 - 53).

2. Procédé selon la revendication 1,
**caractérisé en ce que** la position de l'au moins un élément de référence d'imagerie (32, 54, 55) est déterminée à l'aide de l'emplacement relatif de l'au moins un élément de référence d'imagerie (32, 54, 55) par rapport à l'au moins un premier élément de référence de position (18, 43 - 53).

3. Procédé selon la revendication 1,
**caractérisé en ce qu'**au moins un deuxième élément de référence de position (34, 62) est prévu à un emplacement relatif connu par rapport à l'au moins un élément de référence d'imagerie (35, 63) et que la position de l'au moins un élément de référence d'imagerie (35, 63) est déterminée par le biais d'une position de l'au moins un deuxième élément de référence de position (34, 62) déterminée par le système de détection de position (16, 82, 86).

4. Procédé selon la revendication 1,
**caractérisé en ce que** l'au moins un premier élément de référence de position et l'au moins un élément de référence d'imagerie sont réalisés en tant qu'au moins un élément de référence combine (36) représentable par le système d'imagerie et détectable par le système de détection de position et la position de l'au moins un élément de référence combine (36) est déterminée par le biais du système de détection de position (16, 82, 86).

5. Procédé selon l'une des revendications 1 à 4,
**caractérisé en ce que** le système de détection de position (16, 82, 86) détermine au moins une information sur l'orientation de l'au moins un élément de référence de position (18, 34, 43 - 53, 62, 66, 67, 71, 72, 78) dans l'espace.

6. Procédé selon l'une des revendications 1 à 5,
**caractérisé en ce que** le système d'imagerie (11, 81) détermine au moins une information sur l'orientation de l'au moins un élément de référence d'imagerie (32, 35, 54, 55, 63, 68, 69, 73, 74) par rapport au système d'imagerie (11, 81).

7. Procédé selon l'une des revendications 1 à 6,
**caractérisé en ce que** le système de détection de position (16, 82, 86) comprend au moins un dispositif de prise de vue (17, 77, 83, 87) et que les éléments de référence de position (18, 34, 43 - 53, 62, 66, 67, 71, 72, 78) sont des repères photogrammétriques.

8. Procédé selon la revendication 7,
**caractérisé en ce que** le calibrage du système de détection de position (16, 82, 86) est effectué au moyen d'un système de suivi laser (41).

9. Procédé selon la revendication 8,
**caractérisé en ce que** le logement de patient (3) possède au moins un logement (37) pour un réflecteur (38) du système de suivi laser (41), dans lequel un réflecteur (38) est agencé dans le logement (37)pour la détermination de la position de l'au moins un dispositif de prise de vue (17, 77, 83, 87), et dans au moins une position du logement de patient (3) la position du réflecteur (38) est mesurée au moyen d'un suiveur laser (42) aussi bien que l'agencement de l'au moins un premier élément de référence de position (18, 43 - 53) est enregistré avec l'au moins un dispositif de prise de vue (17, 77, 83, 87).

10. Procédé selon la revendication 8 ou 9,
**caractérisé en ce que** un élément de calibrage (61) est agencé en le logement de patient (3) pour le montage du dispositif d'irradiation de patient (1), dans lequel l'élément de calibrage (61) présente au moins un deuxième élément de référence de position (62), l'au moins un élément de référence d'imagerie (63) détectable par le système d'imagerie (11, 81) et au moins un réflecteur (64) du système de suivi laser (41), dans lequel l'agencement du deuxième élément de référence de position (62), de l'élément de référence d'imagerie (63) et du réflecteur (64) en l'élément de calibrage (61) est connu et que la position du réflecteur (64) est déterminée dans l'espace au moyen d'un suiveur laser (42), l'agencement de l'au moins un deuxième élément de référence de position (62) est enregistré avec l'au moins un dispositif de prise de vue (17, 77, 83, 87) et la position de l'élément de référence d'imagerie (63) est détectée avec le système d'imagerie (11, 81) et que la position de l'au moins un deuxième élément de référence de position (62), au moins une information sur la position de la représentation générée avec le système d'imagerie (11, 81) de l'élément de référence d'imagerie (63) et la position du dispositif de prise de vue (17, 77, 83, 87) sont déterminées à partir de là.

11. Procédé selon l'une des revendications 7 à 10,
**caractérisé en ce que** la détermination de la position du logement de patient (3) est effectuée par le biais d'un champ de points de référence, qui est généré à partir d'au moins une image de l'au moins un élément de référence de position (18, 35, 43 - 53, 62) enregistrée par l'au moins un dispositif de prise de vue (17, 77, 83, 87).

12. Procédé selon l'une des revendications 7 à 11,
**caractérisé en ce que** pour le montage du dispositif d'irradiation de patient (1), au moins deux éléments de calibrage (56, 57, 65, 70) de poids différent sont agencés l'un après l'autre en le logement de patient (3), dans lequel pour chaque élément de calibrage (56, 57, 65, 70), l'emplacement relatif d'au moins deux éléments de reference (47, 49, 54, 55, 66 - 69, 71 -74) est déterminé l'un par rapport à l'autre et associé à un poids de patient correspondant au poids de l'élément de calibrage (56, 57, 65, 70), dans lequel les au moins deux éléments de reference (47, 49, 54, 55, 66 - 69, 71 -74) sont en particulier au moins deux éléments de référence de position (47, 49, 66, 67, 71, 72) ou au moins deux éléments de référence d'imagerie (54, 55, 68, 69, 73, 74).

13. Procédé selon la revendication 12,
**caractérisé en ce que** les au moins deux éléments de reference (66, 67, 68, 69, 71, 72, 73, 74) sont agencés en l'élément de calibrage (65, 70).

14. Procédé selon la revendication 12,
**caractérisé en ce que** les au moins deux éléments de reference (47, 49, 54, 55) sont agencés en le logement de patient (3).

15. Procédé selon l'une des revendications 7 à 14,
**caractérisé en ce que** le système de détection de position (16, 82, 86) comprend au moins une source de lumière (75, 76), qui éclaire l'au moins un élément de référence de position (18, 34, 43 - 53, 62, 66, 67, 71, 72, 78) au moins pendant un enregistrement.

16. Procédé selon la revendication 15,
**caractérisé en ce que** la source de lumière (75, 76) génère de la lumière visible ou de la lumière infrarouge.

17. Procédé selon l'une des revendications 1 à 16,
**caractérisé en ce que** le système d'imagerie (81) est maintenu mobile en le logement de patient (3).

18. Procédé selon l'une des revendications 1 à 16,
**caractérisé en ce que** le système d'imagerie (11) est mobile dans l'espace indépendamment du logement de patient (3).
